# EUROPEAN PATENT SPECIFICATION

(11) **EP 4 358 008 B1**
(45) Date of publication and mention of the grant of the patent: **07.05.2025**
(21) Application number: 22858094.0
(22) Date of filing: 18.03.2022
(51) Int. Cl.: G06Q 50/02

(54) **VEGETATION MANAGEMENT SYSTEM AND VEGETATION MANAGEMENT METHOD**
VEGETATIONSVERWALTUNGSSYSTEM UND VEGETATIONSVERWALTUNGSVERFAHREN
SYSTÈME DE GESTION DE VÉGÉTATION ET PROCÉDÉ DE GESTION DE VÉGÉTATION

(30) Priority: 19.08.2021 JP 2021134011
(43) Date of publication of application: 24.04.2024
(73) Proprietor: Hitachi Energy Ltd, 8050 Zürich (CH)
(72) Inventor: ZHAO, Yu, Tokyo 100-8280 (JP)
(74) Representative: AWA Sweden AB
(86) International application number: PCT/JP2022/012910
(87) International publication number: WO 2023/021769

(56) References cited:
- JP-A- 2008 079 549
- JP-A- 2011 010 479
- JP-A- 2021 006 017
- JP-B1- 6 806 397
- JP-B1- 6 906 824
- US-A1- 2018 098 137
- US-A1- 2020 235 559

## Description

### TECHNICAL FIELD

The present invention relates to a vegetation management system and a vegetation management method and is suitably applied to a vegetation management system and a vegetation management method for supporting maintenance work for a power facility against contact of vegetation using measurement information of remote sensing.

### BACKGROUND ART

In conventional power facility maintenance work, routes on which power facilities such as distribution lines and power transmission lines are disposed have been periodically investigated by manpower and work such as removal of tree branches and use of a herbicide has been performed for places where problems such as contact of trees with the power facilities are likely to occur. In contrast, in recent years, an approach for automating investigation has been advanced because of labor shortage and the like.

In this approach for the automation of the investigation work, a remote sensing technology that can remotely monitor the power facilities and trees (vegetation) considering that most of the power facilities such as the distribution lines and the power transmission lines are installed in places where it is difficult for people to enter such as mountainous areas has been attracting attention. As representative means of the remote sensing technology, there is use of an artificial satellite, an airplane, a drone, and the like. Further, as a method of determining contact of vegetation and a facility, research and development of vegetation contact determination by three-dimensional measurement utilizing a LiDAR (Light Detection and Ranging) sensor has been advanced.

For example, Patent Literature 1 discloses a system that analyzes growth of plants based on remote sensing images photographed by the remote sensing technology.

### CITATION LIST

### PATENT LITERATURE

PTL 1: Japanese Patent Laying-Open No. 2016-123369

Document JP 6 906824 B1 discloses predicting the growth rates of trees planted in a predetermined area by means of an acquisition unit.

Document JP 2021 006017 A relates to a remote sensing image acquisition timing determination system and crop growth status analysis method.

### SUMMARY OF INVENTION

### TECHNICAL PROBLEM

However, in the related art, when contact of a facility and vegetation is analyzed using remote sensing data, it is necessary to perform vegetation contact determination by three-dimensional measurement utilizing a remote sensing sensor such as a LiDAR sensor. There is a problem in that cost extremely increases because a photographing range is expanded and photographing is performed frequently in order to perform accurate determination. In order to support a user in visualization and intuitive operation, conversion of the facility and the vegetation into three dimensions is an indispensable process. Therefore, a large amount of heterogeneous and time sequence geographical information is necessary, which is also a factor of the increase in cost.

The present invention has been devised in view of the above points and proposes a vegetation management system and a vegetation management method capable of suppressing an increase in cost and accurately performing an analysis of a risk that vegetation and a facility come into contact.

### SOLUTION TO PROBLEM

In order to solve such a problem, the present invention provides a vegetation management system that analyzes, using remote sensing data obtained by photographing, with remote sensing, a facility and vegetation that are analysis targets, a risk that the vegetation comes into contact with the facility, the vegetation management system including: a vegetation classification unit that classifies the vegetation photographed in the remote sensing data; a long-term change prediction unit that predicts, based on a classification result of the vegetation by the vegetation classification unit and the remote sensing data, long-term wide-area fluctuation that is range fluctuation of the vegetation in a predetermined long-term time sequence; a short-term change prediction unit that predicts, based on the classification result of the vegetation by the vegetation classification unit and the remote sensing data, short-term wide-area fluctuation that is range fluctuation of the vegetation in a predetermined short-term time sequence; a risk determination unit that determines, based on a prediction result by the long-term change prediction unit and a prediction result by the short-term change prediction unit, a risk due to contact of the vegetation and the facility; and a visualization unit that visualizes a determination result of the risk by the risk determination unit.

In order to solve such a problem, the present invention provides a vegetation management method by a vegetation management system that analyzes, using remote sensing data obtained by photographing, with remote sensing, a facility and vegetation that are analysis targets, a risk that the vegetation comes into contact with the facility, the vegetation management method including: a vegetation classification step in which the vegetation management system classifies the vegetation photographed in the remote sensing data; a long-term change prediction step in which the vegetation management system predicts, based on a classification result of the vegetation by the vegetation classification step and the remote sensing data, long-term wide-area fluctuation that is range fluctuation of the vegetation in a predetermined long-term time sequence; a short-term change prediction step in which the vegetation management system predicts, based on the classification result of the vegetation by the vegetation classification step and the remote sensing data, short-term wide-area fluctuation that is range fluctuation of the vegetation in a predetermined short-term time sequence; a risk determination step in which the vegetation management system determines, based on a prediction result by the long-term change prediction step and a prediction result by the short-term change prediction step, a risk due to contact of the vegetation and the facility; and a visualization step in which the vegetation management system visualizes a determination result of the risk by the risk determination step.

### ADVANTAGEOUS EFFECTS OF INVENTION

According to the present invention, it is possible to suppress an increase in cost and accurately perform an analysis of a risk that vegetation and a facility come into contact.

### BRIEF DESCRIPTION OF DRAWINGS

Fig. 1 is a block diagram showing a configuration example of a vegetation management system 1 according to an embodiment of the present invention.
Fig. 2 is a block diagram showing a hardware configuration example of vegetation management system 1.
Fig. 3 is a flowchart showing a processing procedure of overall processing in vegetation management system 1.
Fig. 4 is a diagram for explaining an image of data stored in a database 20.
Fig. 5 is a flowchart showing a processing procedure example of vegetation classification processing.
Fig. 6 is a diagram for explaining an example of a vegetation classification map.
Fig. 7 is a flowchart showing a processing procedure example of tree height estimation processing.
Fig. 8 is a diagram for explaining an example of a tree height map.
Fig. 9 is a flowchart showing a processing procedure example of long-term change prediction processing.
Fig. 10 is a diagram for explaining a generation image of a long-term vegetation wide-area map and a long-term tree height prediction map.
Fig. 11 is a flowchart showing a processing procedure example of short-term change prediction processing.
Fig. 12 is a diagram for explaining a generation image of a short-term vegetation wide-area map and a short-term tree height prediction map.
Fig. 13 is a flowchart showing a processing procedure example of risk determination processing.
Fig. 14 is a diagram for explaining an example of growth risk determination by a growth risk model.
Fig. 15 is a diagram for explaining an example of contact risk determination by a contact risk model.
Fig. 16 is a diagram for explaining an example of damage risk determination by a damage simulation.
Fig. 17 is a flowchart showing a processing procedure example of visualization processing.
Fig. 18 is a diagram showing an image example of visualization.
Fig. 19 is a flowchart showing a processing procedure example of maintenance instruction processing.
Fig. 20 is a diagram showing a display output example of a maintenance instruction.
Fig. 21 is a diagram showing an example of a maintenance support screen.

### DESCRIPTION OF EMBODIMENT

An embodiment of the present invention is explained in detail below with reference to the drawings.

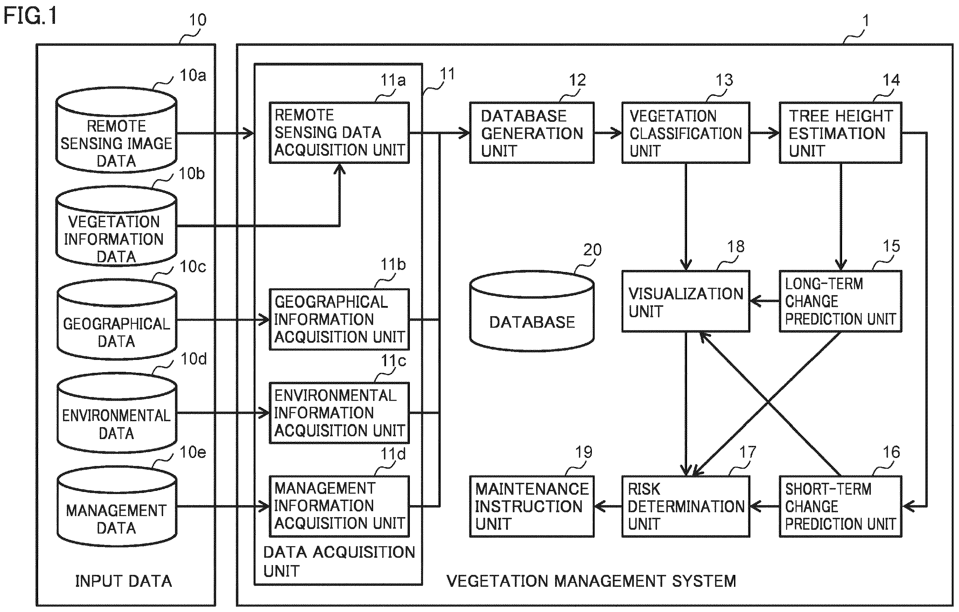

Fig. 1 is a block diagram showing a configuration example of a vegetation management system 1 according to an embodiment of the present invention. Vegetation management system 1 according to this embodiment is a vegetation management system for power facility maintenance that analyzes, for an analysis target region (a target region) designated from a user, a contact risk of vegetation and power facilities using remote sensing data.

As shown in Fig. 1, vegetation management system 1 is a system to which a heterogeneous plurality of input data 10 (remote sensing image data 10a, vegetation information data 10b, geographical data 10c, environmental data 10d, and management data 10e) are input. Vegetation management system 1 includes a data acquisition unit 11 (a remote sensing data acquisition unit 11a, a geographical information acquisition unit 11b, an environmental information acquisition unit 11c, and a management information acquisition unit 11d), a database generation unit 12, a vegetation classification unit 13, a tree height estimation unit 14, a long-term change prediction unit 15, a short-term change prediction unit 16, a risk determination unit 17, a visualization unit 18, a maintenance instruction unit 19, and a database 20. Details of the units are explained as appropriate in the following explanation. Note that, in Fig. 1, movement of data and transition of processing are represented by solid lines with arrows. However, since database 20 is accessed from almost all the components, display of arrows is omitted.

Individual data configuring input data 10 are explained.

Remote sensing image data 10a is time sequence image data obtained by a remote sensing sensor observing the ground surface and specifically is a satellite photograph or an aerial photograph by, for example, RapidEye, MODIS (Moderate Resolution Imaging Spectroradiometer), LANDSAT, Sentinel, or ICESat (Ice, Cloud, and Land Elevation Satellite). As remote sensing image data 10a, for example, a time sequence low-resolution remote sensing image and a high-resolution remote sensing image can be used. However, in this embodiment, types of data of remote sensing image data 10a are not limited to these types. For example, a time sequence image in the same mote sensing image may be used. Note that, in this explanation, a level of resolution is not determined by an absolute standard but determined by relative comparison.

Vegetation information data 10b is data acquired concerning physiological characteristics of vegetation. A type of the data is not limited but is, for example, vegetation distribution information, spectral information, vegetation type information, and tree height (tree crown height) information, and the like.

Among the data explained above, the vegetation distribution information is information indicating latitude and longitude, a position of a distribution shape, and the like. Spectral data is data of a spectral library obtained by an optical sensor (in particular, a passive sensor that obtains information with light emitted from a target object) observing vegetation. The passive sensor basically has the same structure and the same functions as the structure and the functions of an eye (a naked eye). Specifically, an optical system (equivalent to the crystalline lens of the eye) such as a lens collects light from the target object, forms an image on a detection system (equivalent to the retina of the eye), and obtains spectral or spatial information (for example, a color and a shape). In particular, about a spectrum (a color), whereas the eye captures only a visible ray, the optical sensor can detect a visible ray to an infrared ray in a wide range of a wavelength region. Consequently, a large number of beneficial information that is unrecognizable to the eye, such as identification of mineral/rock and vegetation, temperature on the ground surface, a situation of use of a land, and water resources, plankton resources, and the like in the sea and lakes and marshes can be obtained. Furthermore, the optical sensor can also obtain these kinds of beneficial information as two-dimensional images over a wide range. The vegetation type information and tree height information can be obtained by site sampling and actual measurement.

Geographical data 10c is data of geographical information provided from a geographical information system (GIS) and includes information concerning positions and shapes (for example, polygon) of power facilities (distribution lines, power transmission lines, and the like) and vegetation. Geographical data 10c is data indispensable when data acquired in the site and data acquired from a satellite, an airplane, and the like are shared in vegetation management system 1.

Environmental data 10d is data concerning an environment of the site (a region). Examples of environmental data 10d include soil data, meteorological data, altitude data, and inclination angle data in an analysis target region. Examples of the meteorological data specifically include data of AMeDAS, MODIS ground surface temperature data, and meteorological office data but are not limited to these.

Management data 10e is data referred to when content of a maintenance instruction by maintenance instruction unit 19 is determined and includes information concerning management and maintenance of the power facilities. Specifically, for example, management data 10e is data in operation such as an operation maintenance memorandum, a log, and a history of a power company and is provided from the power company.

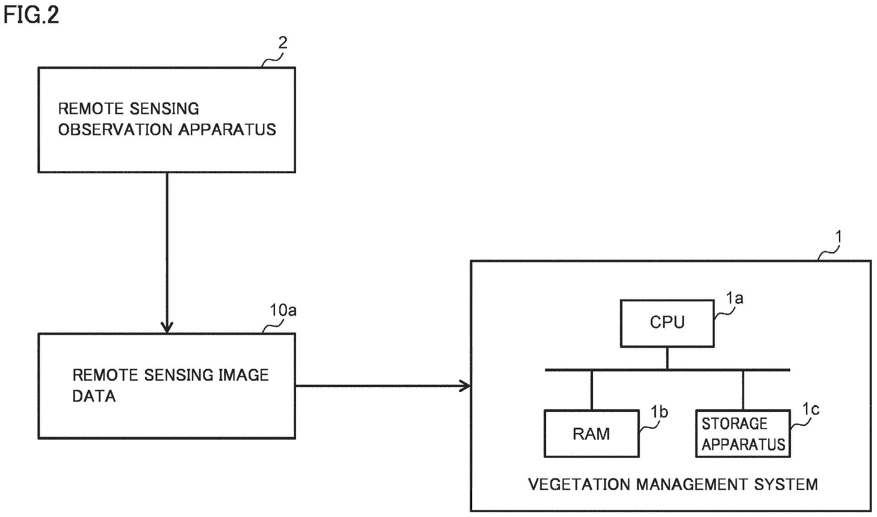

Fig. 2 is a block diagram showing a hardware configuration example of vegetation management system 1. In Fig. 2, a state in which remote sensing image data 10a provided from a remote sensing observation apparatus 2 is input to vegetation management system 1 is shown.

In Fig. 2, remote sensing observation apparatus 2 is an observation satellite, an airplane, or the like including a remote sensing sensor (a LiDAR or the like) and a type of remote sensing observation apparatus 2 is not limited. Remote sensing observation apparatus 2 periodically provides remote sensing image data 10a as an observation result by the remote sensing sensor. Vegetation management system 1 acquires remote sensing image data 10a as one of input data 10. In the following explanation of this embodiment, a satellite image obtained by the observation satellite is used as remote sensing image data 10a.

Vegetation management system 1 can be realized by a general computer system (PC) having an arithmetic function. For example, as shown in Fig. 2, vegetation management system 1 includes a CPU (Central Processing Unit) 1a, a RAM (Random Access Memory) 1b, and a storage apparatus 1c. Note that vegetation management system 1 may include components other than the components shown in Fig. 2. It is conceivable that vegetation management system 1 includes, for example, an output apparatus such as a display as an output destination of a maintenance instruction.

In vegetation management system 1, the functional units shown in Fig. 1 (data acquisition unit 11, database generation unit 12, vegetation classification unit 13, tree height estimation unit 14, long-term change prediction unit 15, short-term change prediction unit 16, risk determination unit 17, visualization unit 18, and maintenance instruction unit 19) are implemented as a plurality of combinations of CPU 1a and RAM 1b divided for each of roles in order to respectively perform arithmetic processing. These functional units can perform readout/writing of data by accessing storage apparatus 1c. Database 20 of vegetation management system 1 shown in Fig. 1 is implemented by storage apparatus 1c. Storage apparatus 1c can include, specifically, for example, not only a storage medium such as an HDD (Hard Disk Drive) or an SSD (Solid State Drive) incorporated in a PC but also a storage medium such as a USB (Universal Serial Bus) memory externally connected to the PC. Storage means such as a database communicably connected to the PC via a network may be included in an example of storage apparatus 1c.

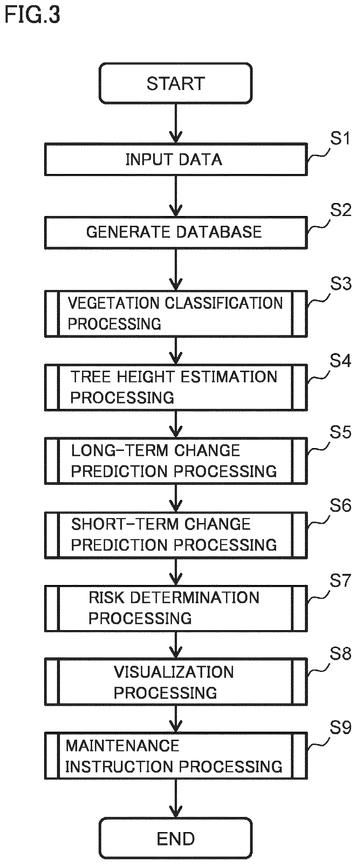

Fig. 3 is a flowchart showing a processing procedure of overall processing in vegetation management system 1. Note that, prior to a start of the processing shown in Fig. 3, an analysis target region (a target region) is designated to vegetation management system 1 from the user.

First, in step S1, an input of input data 10 to vegetation management system 1 is performed by data acquisition unit 11. When explained in individual components of data acquisition unit 11, remote sensing data acquisition unit 11a acquires remote sensing image data 10a and vegetation information data 10b, geographical information acquisition unit 11b acquires geographical data 10c, and environmental information acquisition unit 11c acquires environmental data 10d. In this embodiment, environmental data 10d includes meteorological data concerning temperature, precipitation, sunshine, or the like but may be other kinds of data. Management information acquisition unit 11d acquires management data 10e. In this embodiment, management data 10e includes a monitoring log, a wood cutting log, a maintenance place, and a time history but may be other kinds of data. Input data 10 acquired by data acquisition unit 11 in step S1 is transmitted to database generation unit 12.

In the next step S2, database generation unit 12 stores, in database 20, input data 10 transmitted from data acquisition unit 11 in step S1 and performs data generation processing explained below and stores data after the processing in database 20.

The data generation processing is mainly processing for preparing intermediate data necessary to perform processing by units explained below. Specifically, processing explained below is performed. In the data generation processing, first, database generation unit 12 performs, using information concerning positions and shapes included in geographical data 10c, on remote sensing image data 10a (for example, a satellite image) of a time sequence in the past, masking of the target region designated from the user. Further, database generation unit 12 combines "remote sensing data" obtained by extracting a target region, which is a masking part, from remote sensing image data 10a and "geographical data" obtained by extracting information concerning the target region from geographical data 10c and generates mapping data of the target region. Note that, in this example, it is assumed that the remote sensing data to be extracted includes at least remote sensing data indicating a digital surface model. Database generation unit 12 performs the masking of the target region on vegetation information data 10b and environmental data 10d as well and generates "vegetation information data" and "environmental data" obtained by extracting information concerning the target region. Database generation unit 12 may perform the masking of the target region on management data 10e as well and extract "management data" obtained by extracting information concerning the target region. Database generation unit 12 stores the generated various data in database 20. Further, in step S2, database generation unit 12 combines, besides the data explained above, the remote sensing data, the vegetation information data, the geographical data, and the environmental data stored in database 20 as appropriate, performs mapping, and stores data of the mapping in database 20.

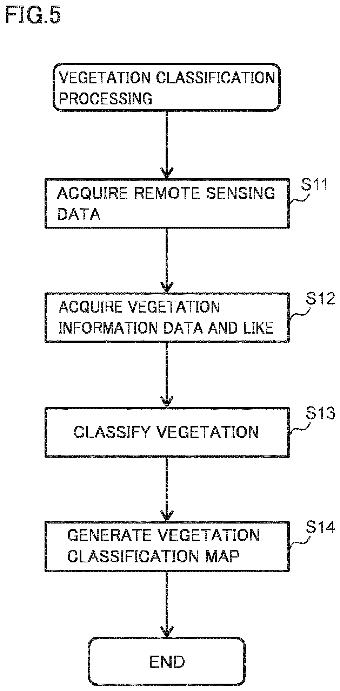

In the next step S3, vegetation classification unit 13 discriminates a type of vegetation based on the remote sensing data and geographical data (or the mapping data combine with the geographical data) and the vegetation information data generated and stored in database 20 in step S2, generates a "vegetation classification map" obtained by reflecting the discriminated type of the vegetation on the mapping data, and stores the "vegetation classification map" in database 20 (vegetation classification processing). Details of the vegetation classification processing are explained below with reference to Fig. 5 and the like.

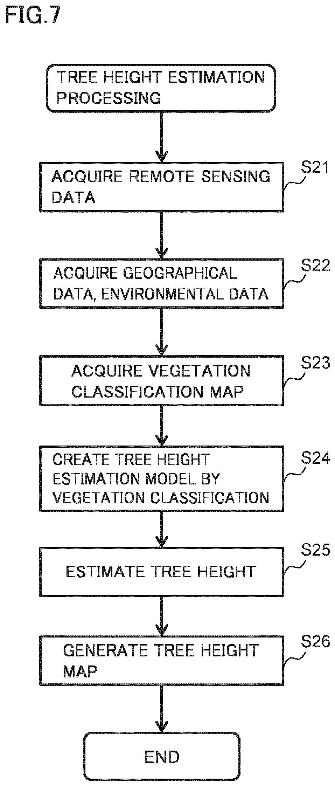

In the next step S4, tree height estimation unit 14 constructs, using the vegetation classification map, the remote sensing data (multi-band satellite image data of the remote sensing data indicating the digital surface model), the geographical data, the environmental data (the meteorological data and the altitude data), and the like stored in database 20, for each kind of vegetation classified in step S3, a tree height estimation model for estimating tree crown height of the vegetation photographed in the remote sensing data, estimates tree heights of respective kinds of vegetation using the model, generates a tree height map obtained by mapping an estimation result on the geographical data, and stores the tree height estimation model and the tree height map in database 20 (tree height estimation processing). Details of the tree height estimation processing are explained below with reference to Fig. 7 and the like.

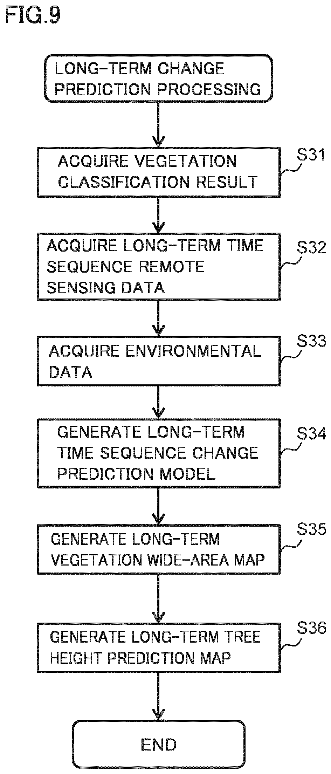

In the next step S5, long-term change prediction unit 15 predicts, using the vegetation classification map, the environmental data, the remote sensing data by a long-term time sequence in the past, and the like stored in database 20, long-term wide-area fluctuation, which is range fluctuation of the vegetation in a predetermined long-term time sequence (for example, designated from the user), further predicts, using the tree height estimation model generated in step S4, long-term tree height fluctuation, which is tree height fluctuation of the vegetation in the predetermined long-term time sequence, and stores a prediction result thereof in database 20 (long-term change prediction processing). Details of the long-term change prediction processing are explained below with reference to Fig. 9 and the like.

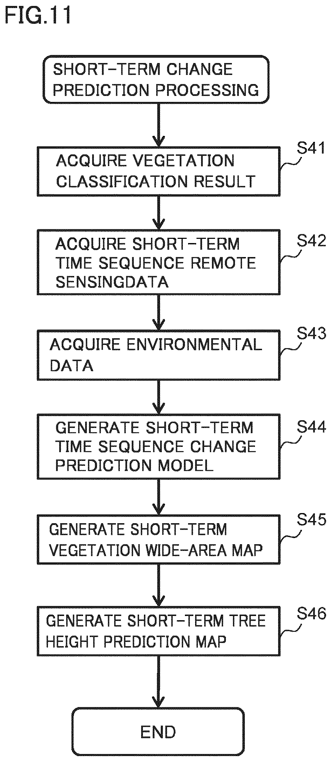

In the next step S6, short-term change prediction unit 16 predicts, using the vegetation classification map, the environmental data, the remote sensing data by a short-term time sequence in the past, and the like stored in database 20, short-term wide-area fluctuation, which is range fluctuation of the vegetation in a predetermined short-term time sequence (for example, designated from the user), further predicts, using the tree height estimation model generated in step S4, short-term tree height fluctuation, which is tree height fluctuation of the vegetation in the predetermined short-term time sequence, and stores a prediction result thereof in database 20 (short-term change prediction processing). Details of the short-term change prediction processing are explained below with reference to Fig. 11 and the like.

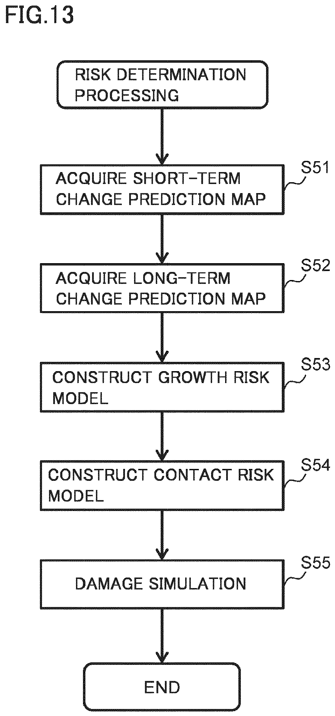

In the next step S7, risk determination unit 17 determines (calculates), based on the prediction results of the long-term/short-term wide-area fluctuation and tree-height fluctuation by long-term change prediction unit 15 and short-term change prediction unit 16, the tree height map generated by tree height estimation unit 14, and the like, a contact risk of the vegetation and the power facilities involved in the time sequence fluctuation and stores a determination result (a risk determination result) in database 20 (risk determination processing). In the risk determination processing, first, risk determination unit 17 maps, on the remote sensing data, the long-term/short-term vegetation wide-area maps, the tree height map, and the geographical data of the power facilities stored in database 20. Subsequently, risk determination unit 17 evaluates a two-dimensional positional relation between the time sequence fluctuation of the vegetation wide-area map and the power facilities and evaluates a three-dimensional positional relation between the time sequence fluctuation of the vegetation tree height and the power facilities. Then, risk determination unit 17 determines, based on results of both the evaluations, a growth risk of the vegetation, contact risk of the vegetation and the power facilities, and a damage risk of the power facilities, and stores a determination result in database 20. Details of the risk determination processing are explained below with reference to Fig. 13 and the like.

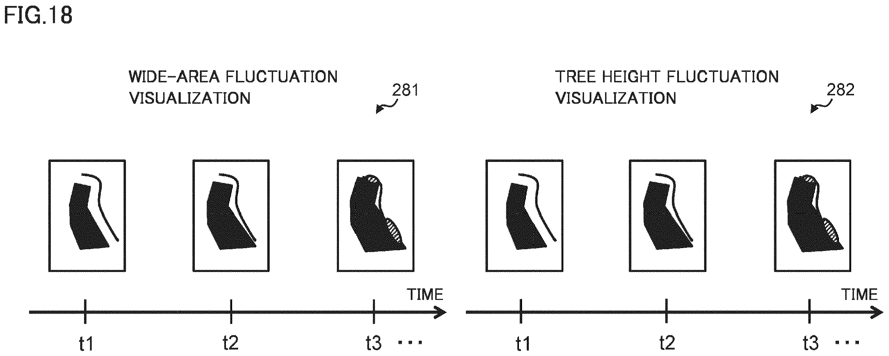

In the next step S8, visualization unit 18 performs, using the prediction results of the wide-area fluctuation and the tree height fluctuation and the risk determination result stored in database 20, two-dimensional or three-dimensional visualization at a time interval of a specific time (or a designated time by the user) (visualization processing). Details of the visualization processing are explained below with reference to Fig. 18.

In the last step S9, maintenance instruction unit 19 presents a maintenance instruction to the user based on an analysis result and a visualization result up to step S8 and management data 10e (or management data) stored in database 20 (maintenance instruction processing). Details of the maintenance instruction processing are explained below with reference to Fig. 18.

Since the processing in steps S1 to S9 explained above is performed, for the target region designated from the user, using the remote sensing data, vegetation management system 1 can predict fluctuation over a long term and a short term of the vegetation at low cost and accurately even if the target region is a wide range, analyze a contact risk of the power facilities and the vegetation, and instruct maintenance work to the user based on a result of the analysis. Since the various data (the input data, the intermediate data, and the output data) input or generated in the series of analysis explained above are stored in database 20, vegetation management system 1 can use these data for subsequent analyses and other analyses.

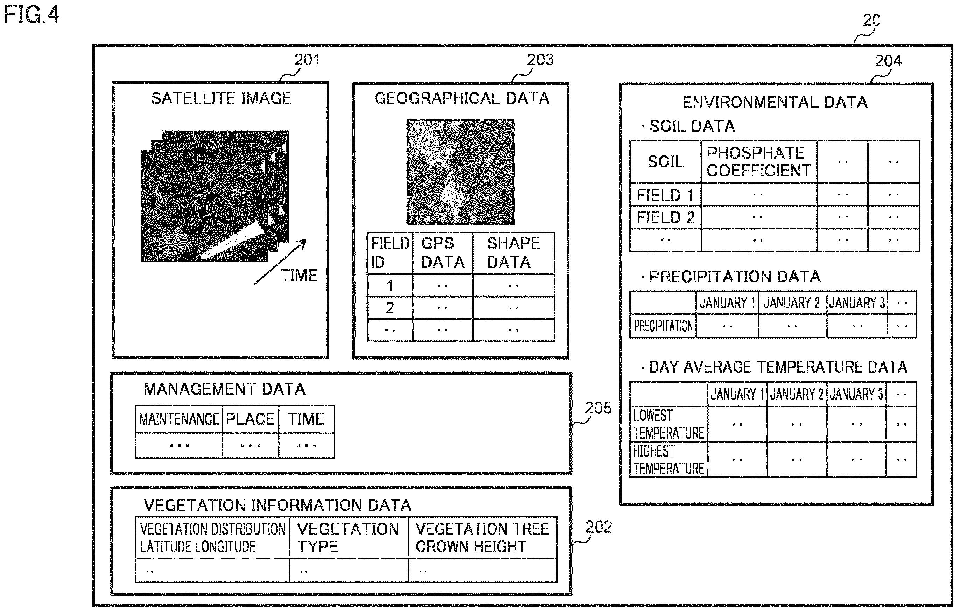

Fig. 4 is a diagram for explaining an image of data stored in database 20. In Fig. 4, as an example of data used as the intermediate data, for a satellite image 201, vegetation information data 202, geographical data 203, environmental data 204, and management data 205, which are remote sensing data, images of the respective data are visually shown.

As explained above in step S2, satellite image 201 is an example of remote sensing image data 10a in the time sequence from which the target region is extracted by database generation unit 12. Similarly, vegetation information data 202 is an example of vegetation information data 10b from which the information concerning the target region is extracted, geographical data 203 is an example of geographical data 10c from which the information concerning the target region is extracted, environmental data 204 is an example of environmental data 10d from which the information concerning the target region is extracted, and management data 205 is an example of management data 10e from which the information concerning the target region is extracted. However, in Fig. 4, illustration of detailed contents of the respective kinds of information is omitted for simplicity.

Fig. 5 is a flowchart showing a processing procedure example of the vegetation classification processing. As explained above, the vegetation classification processing is equivalent to the processing in step S3 in Fig. 3 and is executed by vegetation classification unit 13.

According to Fig. 5, first, vegetation classification unit 13 acquires the mapping data of the target region generated by database generation unit 12 combining the remote sensing data and the geographical data in step S2 in Fig. 3 (step S11). Subsequently, vegetation classification unit 13 acquires that mapping data obtained by combining the environmental data, the vegetation information data, and the geographical data generated in step S2 in Fig. 3 (step S12). The processing in steps S11 and S12 may be changed in execution order or may be executed in parallel.

Note that, in steps S11 and S12, vegetation classification unit 13 may directly acquire target data from database generation unit 12 or may acquire target data stored in database 20. This is because, in vegetation management system 1 according to this embodiment, basically, all data including the intermediate data are stored in database 20. In processing by other functional units explained below, similarly, desired data may be acquired from a functional unit at a generation source or may be acquired from database 20.

Subsequently, vegetation classification unit 13 discriminates a type of the vegetation in the mapping range using the mapping data, the vegetation information data, and the like of the target region acquired in steps S11 and S12 (step S13). As a specific method of "vegetation classification" for discriminating a type of vegetation, for example, it is possible to learn spectral library data included in the vegetation information data using a method of machine learning and estimate the type from a spectral feature value of the vegetation. Note that the method of the vegetation classification is not limited to the above example and another publicly-known method may be used.

Subsequently, vegetation classification unit 13 records geographical data for each type of the vegetation classified in step S13 to thereby generate a vegetation classification map and provides the vegetation classification map to database 20 (step S14).

Note that the processing in steps S11 to S14 may be repeatedly executed targeting mapping data in the past of a time sequence. As a result, vegetation classification unit 13 can generate a vegetation classification map in the past of the time sequence.

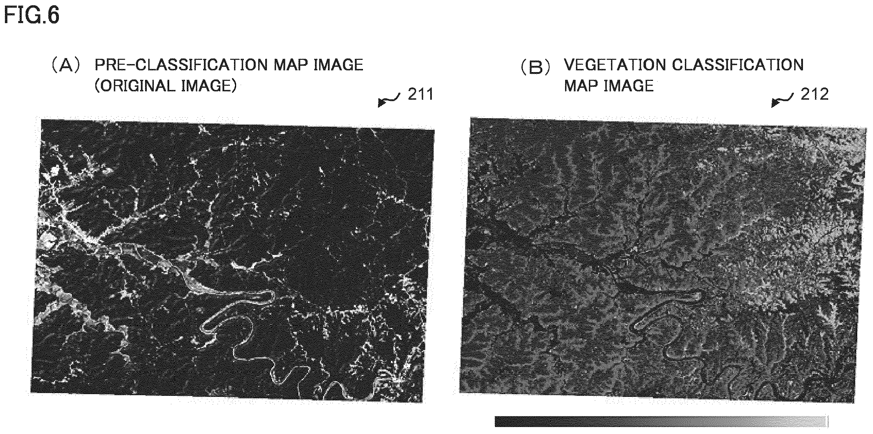

Fig. 6 is a diagram for explaining an example of the vegetation classification map. In Fig. 6(A), a pre-classification map image 211 is shown as an example of mapping data of the target region before the vegetation classification in step S13 in Fig. 5 is performed. In Fig. 6(B), a vegetation classification map image 212 is shown as an example of a vegetation classification map generated in step S14 in Fig. 5 after the vegetation classification. As shown in Fig. 6(A), pre-classification map image 211 is a normal satellite image (remote sensing data). In contrast, vegetation classification map image 212 shown in Fig. 6(B) is a mapping image generated through coloring of pre-classification map image 211 for each type of vegetation through the vegetation classification. Note that, in vegetation classification map image 212, for various kinds of geographical information in the target region, color-coding of ranges and shapes may be performed based on the geographical data.

Fig. 7 is a flowchart showing a processing procedure example of the tree height estimation processing. As explained above, the tree height estimation processing is equivalent to the processing in step S4 in Fig. 3 and is executed by tree height estimation unit 14.

According to Fig. 7, first, tree height estimation unit 14 acquires, from database 20, the multi-band satellite image of the remote sensing data indicating the digital surface model (step S21). Subsequently, tree height estimation unit 14 acquires the geographical data, the environmental data, and the vegetation information data (step S22). Further, tree height estimation unit 14 acquires the vegetation classification map showing the vegetation distribution for each type generated in the vegetation classification processing (step S23). The processing in steps S21 to S23 may be changed in execution order or may be executed in parallel.

Subsequently, tree height estimation unit 14 creates a tree height estimation model for estimating tree crown height for the vegetation classified in the vegetation classification processing based on the data acquired in steps S21 to S23 (step S24). For the creation of the tree height estimation model, a known method may be adopted. For example, a machine learning model of Random forest can be used. In this case, the tree height estimation model can be constructed by using a value of the digital surface model as an objective variable and using the other spectral data, meteorological data, altitude data, vegetation information data, and the like as explanatory variables.

Subsequently, tree height estimation unit 14 estimates height (tree height) of a tree crown for each type of the vegetation using the tree height estimation model constructed in step S24 (step S25). Tree height estimation unit 14 maps an estimation result in step S25 on the geographical map to generate a tree height map and stores the tree height map in database 20 together with the tree height estimation model constructed in step S24 (step S26) and ends the tree height estimation processing.

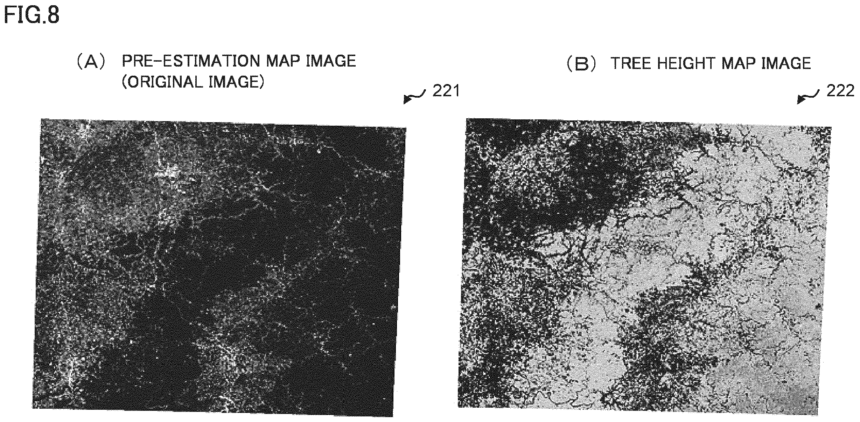

Fig. 8 is a diagram for explaining an example of the tree height map. In Fig. 8(A), a pre-estimation map image 221 is shown as an example of the mapping data of the target region before the tree height estimation processing is performed. In Fig. 8(B), a tree height map image 222 is shown as an example of the tree height map generated in step S26 in Fig. 7 based on a result of the tree height estimation. As shown in Fig. 8(A), pre-estimation map image 221 is a normal satellite image (remote sensing data). In contrast, tree height map image 222 shown in Fig. 8(B) is a mapping image generated through coloring of pre-estimation map image 221 for each of heights of tree crowns estimated using the tree height estimation model for respective kinds of vegetation. Note that, in tree height map image 222, for various kinds of geographical information in the target region, color-coding of ranges and shapes may be performed based on the geographical data.

Fig. 9 is a flowchart showing a processing procedure example of the long-term change prediction processing. As explained above, the long-term change prediction processing is equivalent to the processing in step S5 in Fig. 3 and is executed by long-term change prediction unit 15.

Note that the long-term change prediction processing shown in Fig. 9 is processing for predicting long-term fluctuation of vegetation. "Long term" in this embodiment means a long period in a degree in which changes and fluctuation of the vegetation due to growth of trees and the like can be checked. The "long term" can be considered approximately one year as a unit. In contrast, the short-term change prediction processing shown in Fig. 11 is processing for predicting short-term fluctuation of vegetation. "Short term" in this embodiment means a short period in a degree in which changes and fluctuation of the vegetation due to meteorological fluctuation can be checked such as fall-down of trees due to weather and season changes. The "short term" can be considered approximately three months as a unit.

According to Fig. 9, first, long-term change prediction unit 15 acquires the results of the vegetation classification processing (the tree height estimation model and the vegetation classification map) from database 20 (step S31). Subsequently, long-term change prediction unit 15 acquires, from database 20, the multi-band satellite image of the remote sensing data by the long-term time sequence in the past (step S32). Further, long-term change prediction unit 15 acquires the environmental data, the vegetation information data, and the geographical data from database 20 (step S33). The processing in steps S31 to S33 may be changed in execution order or may be executed in parallel.

Subsequently, long-term change prediction unit 15 generates, based on the long-term time sequence remote sensing data acquired in step S32, a long-term time sequence change prediction model for predicting remote sensing data in long-term future (step S34). The long-term time sequence prediction model can predict a time sequence fluctuation by generating, for example, an RNN (regressive neural network) but may generate and predict another known model.

Subsequently, long-term change prediction unit 15 predicts, using the prediction model generated in step S34, wide-area fluctuation of the vegetation after a long-term designated time (or in every designated time lapse) designated from the user and generates a wide-area map on which a result of the prediction is mapped (step S35). That is, the wide-area map generated in step S35 represents a prediction result of the wide-area fluctuation of the vegetation over a long term. Note that, when the wide-area map is generated, the various data (the vegetation classification map, the environmental data, the vegetation information data, and the geographical data) acquired in steps S31 to S33 may be used as appropriate.

Subsequently, long-term change prediction unit 15 performs, on the wide-area map of the vegetation generated in step S35, tree height estimation using the tree height estimation model for the various kinds of vegetation generated in the tree height estimation processing in Fig. 7 to thereby predict tree height fluctuation of the vegetation after a long-term designated time (or in every designated time lapse) and generates a tree height prediction map on which a result of the prediction is mapped (step S36). Note that, when the tree height prediction map is generated, the various data (the vegetation classification map, the environmental data, the vegetation information data, and the geographical data) acquired in steps S31 to S33 may be used as appropriate.

Note that, in the following explanation, in order to distinguish the wide-area map of the vegetation generated in step S35 of the long-term change prediction processing and a wide-area map of the vegetation generated in step S45 of the short-term change prediction processing explained below, the former is referred to as long-term vegetation wide-area map and the latter is referred to as short-term vegetation wide-area map. Similarly, the tree height prediction map generated in step S36 of the long-term change prediction processing is referred to as long-term tree height prediction map and a tree height prediction map generated in step S46 of the short-term prediction processing is referred to as short-term tree height map.

Finally, long-term change prediction unit 15 stores the prediction results (the long-term vegetation wide-area map and the long-term tree height prediction map) in steps S35 and S36 in database 20 and ends the long-term change prediction processing.

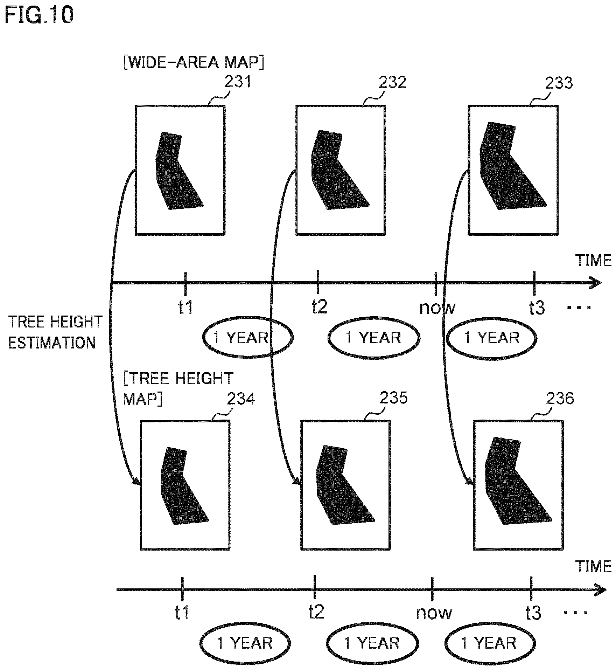

Fig. 10 is a diagram for explaining generation images of the long-term vegetation wide-area map and the long-term tree height prediction map. In Fig. 10, as an example of the long-term time sequence, intervals among times t1 and t2, present (now), and time t3 are set to one year.

As shown in Fig. 10, in the long-term change prediction processing, long-term change prediction unit 15 predicts, from satellite images (wide-area maps 231 and 232) at times t1 and t2 in the past, using the long-term time sequence change prediction model, wide-area fluctuation of the vegetation at a designated time in future (for example, time t3) and generates a wide-area map (a wide-area map 233) on which a prediction result is mapped. Although not illustrated, long-term change prediction unit 15 performs wide-area fluctuation prediction for the vegetation and generates a wide-area map in the same manner for times after time t3. As a result, a wide-area map predicted for the future becomes a long-term vegetation wide-area map representing long-term fluctuation prediction of the vegetation in a wide-area range.

Subsequently, long-term change prediction unit 15 estimates, for each of the wide-area maps (wide-area maps 231 to 233) at the respective times, tree height of the vegetation using the tree height estimation model and generates tree height maps (tree height maps 234 to 236) on which an estimation result is mapped. Among these tree height maps, the tree height map generated for a future time (time t3 and subsequent times) becomes a long-term tree height prediction map representing fluctuation prediction of height of the tree crown in a long-term time sequence.

Fig. 11 is a flowchart showing a processing procedure example of the short-term change prediction processing. As explained above, the short-term change prediction processing is equivalent to the processing in step S6 in Fig. 3 and is executed by short-term change prediction unit 16.

According to Fig. 11, first, short-term change prediction unit 16 acquire the result of the vegetation classification processing (the vegetation classification map) from database 20 (step S41). Subsequently, short-term change prediction unit 16 acquires the multi-band satellite image of the remote sensing data by the short-term time sequence in the past from database 20 (step S42). Further, short-term change prediction unit 16 acquires the environmental data, the vegetation information data, and the geographical data from database 20 (step S43). The processing in steps S41 to S43 may be changed in execution order or may be executed in parallel.

Subsequently, short-term change prediction unit 16 generates, based on the short-term time sequence remote sensing data acquired in step S42, a short-term time sequence change prediction model for predicting remote sensing data in a short-term future (step S44). The short-term time sequence prediction model can predict time sequence fluctuation by generating, for example, an RNN (regressive neural network). However, another known model may be generated to predict time sequence fluctuation.

Subsequently, short-term change prediction unit 16 predicts, using the prediction model generated in step S44, wide-area fluctuation of the vegetation after a short-term designated time (or in every designated time lapse) designated from the user and generates a short-term vegetation wide-area map on which a result of the prediction is mapped (step S45). That is, the short-term vegetation wide-area map represents a prediction result of wide-area fluctuation of the vegetation over a short term. Note that, when the short-term vegetation wide-area map is generated, the various data (the vegetation classification map, the environmental data, the vegetation information data, and the geographical data) acquired in step S41 to S43 may be used as appropriate.

Subsequently, short-term change prediction unit 16 performs, on the short-term vegetation wide-area map generated in step S45, tree height estimation using the tree height estimation model for various kinds of vegetation generated in the tree height estimation processing in Fig. 7 to thereby predict tree height fluctuation of the vegetation after a short-term designated time (or every designated time lapse) and generates a short-term tree height prediction map on which a result of the prediction is mapped (step S46). Note that, when the short-term tree height prediction map is generated, the various data (the vegetation classification map, the environmental data, the vegetation information data, and the geographical data) acquired in steps S41 to S43 may be used as appropriate.

Finally, short-term change prediction unit 16 stores the prediction results (the short-term vegetation wide-area map and the short-term tree height prediction map) in steps S45 and S46 in database 20 and ends the short-term change prediction processing.

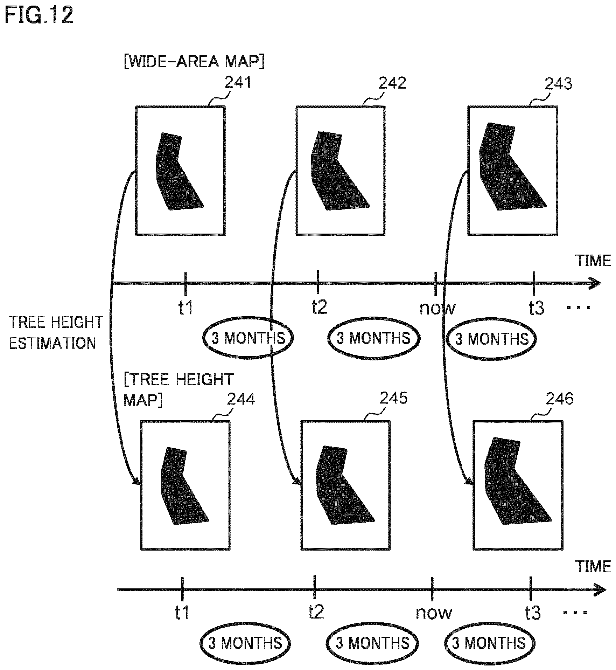

Fig. 12 is a diagram for explaining generation images of the short-term vegetation wide-area map and the short-term tree height prediction map. In Fig. 12, as an example of a short-term time sequence, intervals among times t1 and t2, present (now), and time t3 are set to three months.

As shown in Fig. 12, in the short-term change prediction processing, short-term change prediction unit 16 predicts, from satellite images (wide-area maps 241 and 242) at times t1 and t2 in the past, using the short-term time sequence change prediction model, wide-area fluctuation of the vegetation at a designated time in future (for example, time t3) and generates a wide-area map (a wide-area map 243) on which a prediction result is mapped. Although not illustrated, short-term change prediction unit 16 performs wide-area fluctuation prediction for the vegetation and generates a wide-area map in the same manner for times after time t3. As a result, a wide-area map predicted for the future becomes a short-term vegetation wide-area map representing short-term fluctuation prediction of the vegetation in a wide-area range.

Subsequently, short-term change prediction unit 16 estimates, for each of the wide-area maps (wide-area maps 241 to 243) at the respective times, tree height of the vegetation using the tree height estimation model and generates tree height maps (tree height maps 244 to 246) on which an estimation result is mapped. Among these tree height maps, the tree height map generated for a future time (time t3 and subsequent times) becomes a short-term tree height prediction map representing fluctuation prediction of height of the tree crown in a short-term time sequence.

Fig. 13 is a flowchart showing a processing procedure example of the risk determination processing. As explained above, the risk determination processing is equivalent to the processing in step S7 in Fig. 3 and is executed by risk determination unit 17. Note that, in Fig. 13, the processing in steps S51 and S52 may be changed in execution order or may be executed in parallel.

According to Fig. 13, first, risk determination unit 17 acquires, from, for example, database 20, the short-term vegetation wide-area map and the short-term tree height prediction map generated in the short-term change prediction processing (step S51). Subsequently, risk determination unit 17 acquires, from, for example, database 20, the long-term vegetation wide-area map and the long-term tree height prediction map generated in the long-term change prediction processing (step S52).

Subsequently, risk determination unit 17 constructs, using the short-term vegetation wide-area map and the long-term vegetation wide-area map acquired in steps S51 and S52, a growth risk model for evaluating a wide-area contact risk (growth risk) of the vegetation and the power facilities due to range fluctuation of the vegetation over a short term/a long term (step S53). Specifically, risk determination unit 17 constructs, from the short-term vegetation wide-area map, a short-term growth risk model for estimating a fall-down risk indicating to which degree fall-down of the vegetation (trees and the like) due to meteorological fluctuation such as weather or a season change occurs in a short-term time sequence lapse such as several months and stores the model in database 20. Risk determination unit 17 constructs, from the long-term vegetation wide-area map, a long-term growth risk model for estimating a contact risk due to growth of vegetation (trees and the like) in a long-term time sequence lapse such as one year and stores the model in database 20.

Subsequently, risk determination unit 17 constructs, based on the short-term/long-term growth risks calculated from the growth risk model in step S53, the short-term/long-term tree height prediction maps acquired in steps S51 and S52, and the geographical data concerning the power facilities (for example, the power transmission lines and the distribution lines), a contact risk model for evaluating a detailed contact risk of the vegetation (the trees and the like) and the power facilities due to the tree height fluctuation of the vegetation over the short term/the long term and stores an evaluation result by the model in database 20 (step S54). That is, in the determination of the contact risk by the contact risk model, geographical information of the power facilities and geographical information of boundary lines with the vegetation (the trees and the like) are collated and presence or absence of contact (or a contact place) is determined. A method of constructing the contact risk model is not particularly limited. However, the contact risk model can be constructed using for example, a mathematical model, deep learning, or machine learning.

Subsequently, risk determination unit 17 executes, based on the contact risk calculated in step S54, a damage simulation for predicting a damage risk of the power facilities (damage risk determination), stores a result of the prediction in database 20, and ends the risk determination processing.

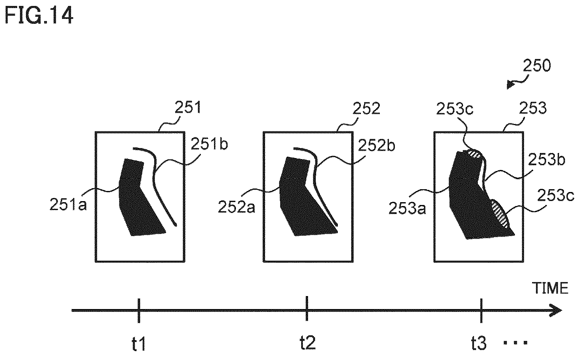

Fig. 14 is a diagram for explaining an example of the growth risk determination by the growth risk model. In Fig. 14, as images of a growth risk model 250 in a time sequence lapse of times t1, t2, and t3 (t3 is future), two-dimensional maps 251, 252, and 253 obtained by two-dimensionally mapping wide-area maps of vegetation and power facilities at the times are shown.

In Fig. 14, in two-dimensional map 251, a vegetation range 251a and a power transmission line 251b (an example of a power facility) are not in contact. In two-dimensional map 252, a vegetation range 252a and a power transmission line 252b are not in contact. On the other hand, in two-dimensional map 253 that is a future prediction model, it is indicated that a vegetation range 253a and a power transmission line 253b are likely to come into contact in a risk spot 253c. From such a growth risk model 250, a growth risk of risk spot 253c is determined at time t3. In the growth risk determination using the growth risk model explained above, determination using the short-term growth risk model and determination using the long-term growth risk model are separately executed. In the short-term growth risk determination, it is possible to determine a short-term growth risk that, for example, trees fall down because of meteorological fluctuation such as weather (rain, typhoon, or the like) and a season change. In the long-term growth risk determination, it is possible to determine a long-term growth risk due to growth of trees.

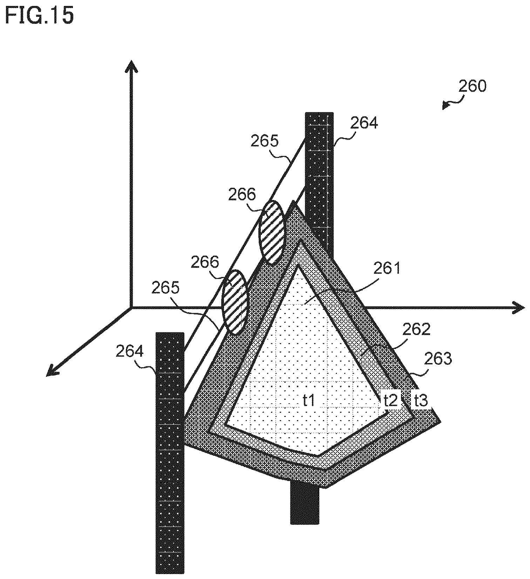

Fig. 15 is a diagram for explaining an example of the contact risk determination by the contact risk model. In Fig. 15, as images of a contact risk model 260 in a time sequence lapse of times t1, t2, and t3 (t3 is future), vegetation and power facilities at the times are three-dimensionally mapped. Individually, tree crown models 261, 262, and 263 indicating tree crowns of the vegetation at the times and power poles 264 and power transmission lines 265, which are examples of power facilities, are shown. Disposition of the power facilities can be generated from the geographical data.

In Fig. 15, tree crown models 261 and 262 at times t1 and t2 are not in contact with all of power poles 264 and power transmission lines 265. On the other hand, it is indicated that tree crown model 263 at time t3, which is a future prediction model, is likely to come into contact with power transmission line 265 in risk spots 266. From such a contact risk model 260, a contact risk of risk spots 266 is determined at time t3.

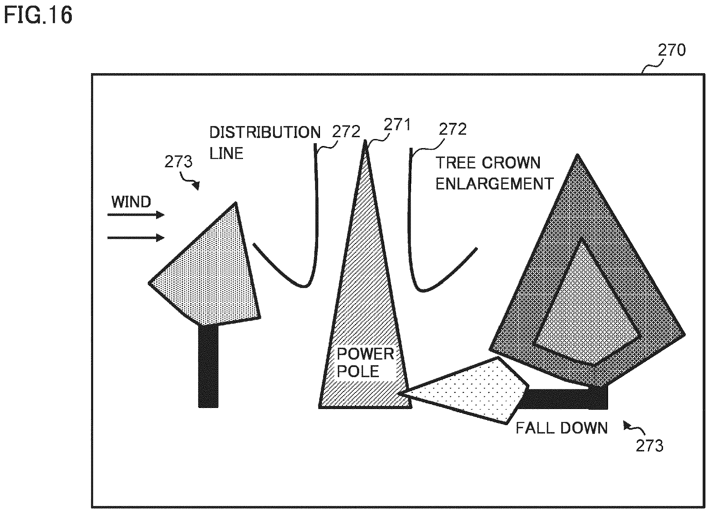

Fig. 16 is a diagram for explaining an example of the damage risk determination by the damage simulation. In Fig. 16, an image diagram of a damage risk model 270 used for the damage simulation is shown. In the damage risk determination, detailed determination is performed for places having a high contact possibility (for example, risk spots 266 in Fig. 15) determined by the contact risk determination. Specifically, as shown in Fig. 16, damage risk model 270 determines, using three-dimensional models of power facilities and vegetation (a power pole 271, distribution lines 272, and vegetation 273), a damage risk of the power facilities due to contact of the vegetation considering the height of trees, wind velocity, fall-down, and expansion of a vegetation amount (in this embodiment, expansion of a tree crown). A method of risk determination is not limited to the above example and known another method may be used.

As explained above, in the risk determination processing shown in Fig. 13, three types of determination including the determination of the short-term/long-term wide-area growth risks (step S53), the determination of the contact risks in the short-term/the long-term (step S54), and the determination of the damage risk in the part where the contact risk is determined (step S55) are executed. Among these determinations, the determination of the growth risk is grasped as processing for predicting a rough "wide-area risk" from a growth model that estimates future. The determination of the contact risk can be grasped as processing for predicting a finer "detailed risk" using the geographical data of the power facilities. The determination of the damage risk can be grasped as processing for predicting a specific damage risk from a degree of contact for a part where the detailed risk is predicted. Therefore, for example, when possibility of contact is roughly predicted by the determination of the growth risk (the wide-area risk) using the two-dimensional model, by performing the determination of the contact risk (the detailed risk) using the three-dimensional model and the damage simulation for a predicted contact part, various kinds of determination in the risk determination processing may be executed by risk determination unit 17 stepwise such as a detailed analysis of a damage risk predicted in the contact part. Note that, in the above case, the three-dimensional model is used in the contact risk determination and the damage simulation in order to perform highly accurate contact determination. However, this is not restrictive and any method can be used as long as a highly accurate determination result is obtained by the method. For example, remote sensing data (a satellite image) more highly accurate than usual may be used.

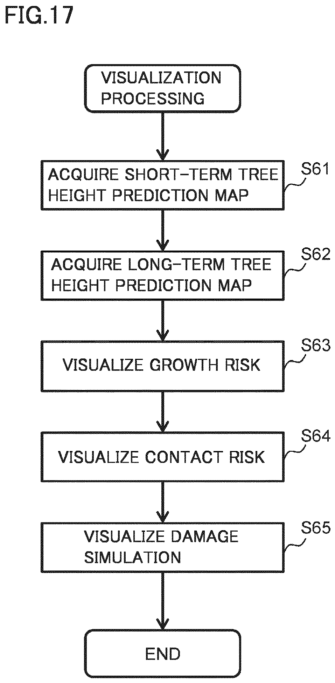

Fig. 17 is a flowchart showing a processing procedure example of the visualization processing. As explained above, the visualization processing is equivalent to the processing in step S8 in Fig. 3 and is executed by visualization unit 18. Note that, in Fig. 17, the processing in steps S61 and S62 may be changed in execution order or may be executed in parallel.

According to Fig. 17, first, visualization unit 18 acquires from, for example, database 20, the short-term vegetation wide-area map and the short-term tree height prediction map generated in the short-term change prediction processing and the geographical data concerning the power facilities (for example, the distribution lines and the power transmission lines) and the vegetation corresponding to these maps (step S61). Subsequently, visualization unit 18 acquires from, for example, database 20, the long-term vegetation wide-area map and the long-term tree height prediction map generated in the long-term change prediction processing and the geographical data concerning the power facilities (for example, the distribution lines and the transmission lines) and the vegetation corresponding to these maps (step S62).

Subsequently, visualization unit 18 performs visualization of the growth risk based on a determination result of the growth risk calculated in step S53 of the risk determination processing (step S63). In the visualization, the data acquired in steps S61 and S62 can be used in combination as appropriate.

Subsequently, visualization unit 18 performs visualization of the contact risk based on the determination result of the contact risk calculated in step S54 of the risk determination processing (step S64). In the visualization, the data acquired in steps S61 and S62 can be used in combination as appropriate.

Subsequently, visualization unit 18 performs visualization of the damage risk based on the determination result of the damage risk calculated in step S55 of the risk determination processing (step S65) and ends the visualization processing. In the visualization, the data acquired in steps S61 and S62 can be used in combination as appropriate.

Fig. 18 is a diagram showing an image example of the visualization. In Fig. 18, an example of the visualization of the growth risk in step S63 in Fig. 17 is shown.

In Fig. 18, a visualization image 281 of wide-area fluctuation and a visualization image 282 of tree height fluctuation are indicated by two-dimensional images for the respective times of designated times t1, t2, and t3. Visualization image 281 of the wide-area fluctuation and visualization image 282 of the tree height fluctuation can be respectively generated from the vegetation wide-area map and the tree height map used in the growth risk determination at the designated times. Note that, although being shown as still images in Fig. 18, the visualization images may be displayed as moving images at designated time resolution during a designated time interval (t1 to 3).

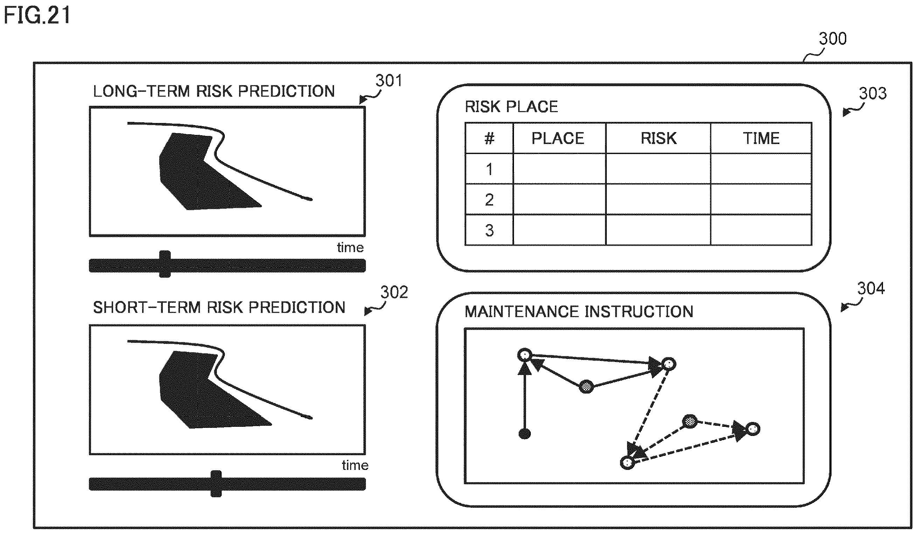

Although specific illustration is omitted, in the visualization of the contact risk in step S64 in Fig. 17, the contact risk of the vegetation and the power facilities at the designated times only has to be displayed using the three-dimensional image illustrated in Fig. 15 (or a moving image). In the visualization of the damage risk in step S65 in Fig. 17, the damage risk of the power facilities due to the contact of the vegetation at the designated times only has to be displayed using the two-dimensional image illustrated in Fig. 16 (or a moving image). A three-dimensional image (or a moving image) can also be used in the visualization of the damage risk. The visualization of the risk determinations in steps S63 to S65 in Fig. 17 may be, for example, collectively displayed on a management screen as shown in Fig. 21 below.

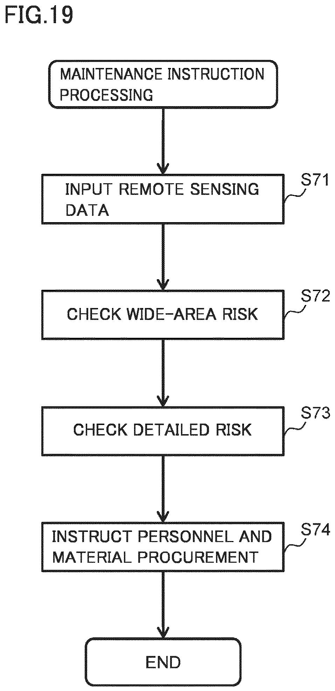

Fig. 19 is a flowchart showing a processing procedure example of the maintenance instruction processing. As explained above, the maintenance instruction processing is equivalent to the processing in step S9 in Fig. 3 and is executed by maintenance instruction unit 19.

According to Fig. 19, first, maintenance instruction unit 19 acquires the remote sensing data from database 20 and inputs the remote sensing data (step S71). Examples of the remote sensing data acquired in step S81 include a satellite image, a drone image, and a vehicle-mounted camera image. However, the remote sensing data may be other data.

Subsequently, maintenance instruction unit 19 checks a determination result of the risk determination processing using the remote sensing data acquired in step S81 (steps S72 and S73).

Specifically, in step S72, maintenance instruction unit 19 checks the contact risk in the wide area. More specifically, maintenance instruction unit 19 checks a risk spot in the wide area (for example, risk spot 253c in Fig. 14) determined in the growth risk determination (step S53 in Fig. 13) in the risk determination processing and a risk state in the wide area (for example, the risk spot shown in visualization image 281 in Fig. 18) visualized in the growth risk visualization (step S63 in Fig. 17) in the visualization processing and selects a region of interest where the contact risk in the wide area is predicted between the power facilities and the vegetation. The region of interest only has to be selected using a threshold based on quantization of a risk value. However, another method may be used.

In step S73, maintenance instruction unit 19 checks a detailed contact risk for the region of interest selected in step S72. Specifically, maintenance instruction unit 19 checks a detailed risk spot (for example, risk spot 266 in Fig. 15) determined in the contact risk determination (step S54 in Fig. 13) in the risk determination processing and a detailed risk situation visualized in the contact risk visualization (step S64 in Fig. 17) in the visualization processing and specifies detailed regions where the contact risk is predicted between the power facilities and the vegetation. The detailed regions only have to be specified using a threshold based on quantization of a risk value. However, another method may be used.

Subsequently, maintenance instruction unit 19 acquires, based on check results of the wide-area risk and the detailed risk checked in steps S72 and S73 and the detailed regions specified in step S73, management data corresponding to the check result and the detailed regions from database 20 and determines instruction content of necessary maintenance work (step S74). For example, specifying of a maintenance place, optimization of car allocation, a business trip schedule of personnel, and conveyance of materials can be included in the instruction content of the maintenance work determined in step S74. A method of determining the instruction content of the maintenance work is not limited to a specific method. For example, specified risk places (the detailed regions) only have to be listed and routes and times for personnel dispatch and material transportation involved in the maintenance work only have to be optimized from registration content of the list. Maintenance instruction unit 19 outputs the determined instruction content of the maintenance work from a predetermined output apparatus (for example, a liquid crystal display or a printer) and ends the maintenance instruction processing.

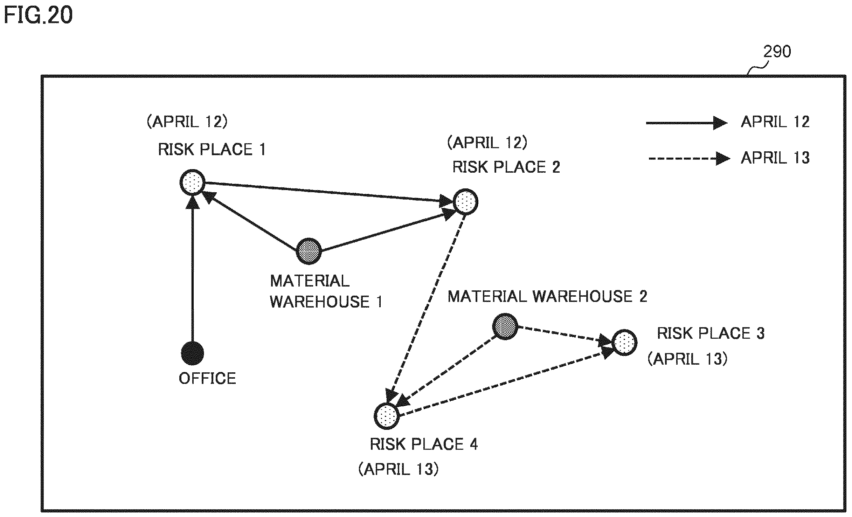

Fig. 20 is a diagram showing a display output example of the maintenance instruction. A maintenance instruction screen 290 shown in Fig. 20 is an output example of the instruction content of the maintenance work output in step S74 in Fig. 19 and is a display screen displayed on a display apparatus (not shown) of vegetation management system 1.

On maintenance instruction screen 290 in Fig. 20, an office, two material warehouses (material warehouses 1 to 2), and four risk places (risk places 1 to 4) are shown. When determining the instruction content of the maintenance work (step S74 in Fig. 19), maintenance instruction unit 19 searches for optimum routes for personnel dispatch and material conveyance with reference to the positions of the office and the warehouses with respect to the risk places. Further, maintenance instruction unit 19 performs calculation considering a moving time and a maintenance work time to thereby estimate maintenance times in the risk places and displays the maintenance times on maintenance instruction screen 290 together with an instruction for routes and times. Specifically, in the case of maintenance instruction screen 290 in Fig. 20, a schedule of movement to the risk places and maintenance work implementation is planned in April 12 or April 13 and routes of personnel dispatch and material transportation are represented by signs with arrows. Note that such instruction content of the maintenance work is different depending on the number and positions of risk places, positions of the office and the warehouses, and the like.

Note that maintenance instruction unit 19 (or visualization unit 18) may display a maintenance support screen concerning the visualization and the maintenance instruction on a GUI (Graphical User Interface) for management or the like and support the maintenance work.

Fig. 21 is a diagram showing an example of the maintenance support screen. A maintenance support screen 300 shown in Fig. 21 is an example of the maintenance support screen displayed as a GUI on a terminal of a maintenance operator and includes a fluctuation display block 301, a fluctuation display block 302, a risk display block 303, and a maintenance instruction block 304. Note that detailed design and detailed items of the GUI is not limited to the example shown in Fig. 21.

Fluctuation display block 301 is a block that displays a prediction result of a growth risk in long-term fluctuation. Fluctuation display block 302 is a block that displays a prediction result of a growth risk in short-term fluctuation. Fluctuation display blocks 301 and 302 make it possible to check a risk spot and a contact situation at a desired designated time with operation of a slide bar provided on a time axis. Risk display block 303 is a block that displays detailed information of a predicted risk and displays a list of risk places and displays information such as latitude and longitude, a risk degree, and a prediction time. Maintenance instruction block 304 is a block that displays instruction content of maintenance work and performs, for example, the same display as the display of maintenance instruction screen 290 illustrated in Fig. 20.

As explained above, with vegetation management system 1 according to this embodiment, it is possible to, using a plurality of remote sensing data capable of photographing a wide area in a time sequence from the past to the present, discriminate a type of vegetation, predict range fluctuation of the vegetation in a long period and a short period, and determine and visualize a risk of contact of the vegetation and a facility (for example, a power facility) based on a result of fluctuation prediction of the vegetation for a long term and a short term. Such a vegetation management system 1 can utilize remote sensing data of two-dimensional data and perform, in two time sequences of a long term/a short term, classification and future fluctuation prediction of the vegetation from measurement data (remote sensing data) in the past, and determine a contact risk with the facility without using three-dimensional data, and visualize the contact risk. Therefore, with vegetation management system 1 according to this embodiment, it is possible to suppress an increase in cost due to use of three-dimensional data and accurately perform an analysis of a risk that the vegetation and the facility come into contact.

Vegetation management system 1 according to this embodiment can predict, based on the remote sensing data, for the vegetation, the type of which is classified, not only fluctuation in the horizontal direction (wide-area fluctuation) of the vegetation but also fluctuation in the vertical direction (tree height fluctuation) of the vegetation by estimating height of a tree crown. Further, since the contact risk with the facility is determined using a result of performing such fluctuation prediction in both of the long-term time sequence and the short-term time sequence, it is possible to realize an accurate and highly precise risk analysis at low cost.

Vegetation management system 1 according to this embodiment can predict, based on the remote sensing data, for the vegetation, the type of which is classified, a change in a vegetation amount in a time or a time interval designated from the user for both of fluctuation in the horizontal direction (wide-area fluctuation) and fluctuation in the vertical direction (tree height fluctuation) of the vegetation. Since a contact risk with the facility can be analyzed, it is possible to provide a system highly convenient for the user.

With vegetation management system 1 according to this embodiment, since the determination results by the growth risk determination and the contact risk determination can be two-dimensionally or three-dimensionally visualized, it is possible to make it easy to visually determine a contact situation of a place having a contact risk.

By performing the instruction of the maintenance work based on the results of the risk determination and the visualization, vegetation management system 1 can cause the user to easily recognize appropriate maintenance work. When determining the work content of the maintenance work, as shown in Fig. 20, when determining the work content of the maintenance work, by integrating information concerning a plurality of risk places and comprehensively making determination, vegetation management system 1 can optimize introduction routes for the personnel and the materials and realize a more efficient instruction of the maintenance work. By displaying a result of the risk determination and the visualization data together as shown in Fig. 21, vegetation management system 1 can clearly inform necessary information to the user.

Vegetation management system 1 according to this embodiment performs the tree height change prediction for the long period and the short period of the vegetation using the data measured in the past and predicts the fluctuation in the wide-area range and the tree height of the vegetation. At this time, by interpolating data used in the tree height prediction and the simulation, vegetation management system 1 can prevent contact of the vegetation and the facility while suppressing a frequency of actually measuring tree height on the site and without excessively using (or without using at all) three-dimensional data for an analysis. As a result, with vegetation management system 1 according to this embodiment, it is possible to expect an effect of substantially reducing inspection cost for the vegetation and the facility. Since the analysis of the vegetation is performed based on the remote sensing data capable of photographing a wide range, it is possible to monitor the vegetation in a wider range than in the past and it is expected that vegetation management system 1 can highly contribute to the inspection and the maintenance of the facility.

Note that the present invention is not limited to the embodiment explained above and includes various modifications. For example, the embodiment explained above is an embodiment explained in detail in order to clearly explain the present invention and is not always limited to an embodiment including all the components explained above. A part of components of a certain embodiment can be replaced with components of another embodiment. Components of another embodiment can be added to components of a certain embodiment. Other components can be added to, deleted from, and replaced with a part of components of an embodiment.

The components, the functions, the processing units, the processing means explained above may be realized by hardware by, for example, designing the part or all thereof with, for example, an integrated circuit. The components, the functions, and the like explained above may be realized by software by a processor interpreting and executing programs for realizing the respective functions. Information of programs, tables, files, and the like for realizing the functions can be stored in a recording device such as a memory, a hard disk, or an SSD or a recording medium such as an IC (Integrated Circuit) card, an SD card, or a DVD (Digital Versatile Disc).

In the drawings, control lines and information lines considered to be necessary in explanation are shown. Not all of control lines and information lines are shown in terms of products. Actually, almost all of the components may be considered to be connected to one another.

### REFERENCE SIGNS LIST

- 1: Vegetation management system
- 1a: CPU
- 1b: RAM
- 1c: Storage apparatus
- 2: Remote sensing observation apparatus
- 10: Input data
- 10a: remote sensing image data
- 10b: Vegetation information data
- 10c: Geographical data
- 10d: Environmental data
- 10e: Management data
- 11: Data acquisition unit
- 11a: Remote sensing data acquisition unit
- 11b: Geographical information acquisition unit
- 11c: Environmental information acquisition unit
- 11d: Management information acquisition unit
- 12: Database generation unit
- 13: Vegetation classification unit
- 14: Tree height estimation unit
- 15: Long-term change prediction unit
- 16: Short-term change prediction unit
- 17: Risk determination unit
- 18: Visualization unit
- 19: Maintenance instruction unit
- 20: Database
- 201: Satellite image
- 202: Vegetation information data
- 203: Geographical data
- 204: Environmental data
- 205: Management data
- 211: Pre-classification map image
- 212: Vegetation classification map image
- 221: Pre-estimation map image
- 222: Tree height map image
- 231 to 233, 241 to 243: Wide-area map
- 234 to 236, 244 to 246: Tree height map
- 250: Growth risk model
- 260: Contact risk model
- 270: Damage risk model
- 281, 282: Visualization image
- 290: Maintenance instruction screen
- 300: Maintenance support screen

## Claims

1. A vegetation management system that analyzes, using remote sensing data obtained by photographing, with remote sensing, a facility and vegetation that are analysis targets, a risk that the vegetation comes into contact with the facility, the vegetation management system comprising:
a vegetation classification unit that classifies the vegetation photographed in the remote sensing data;
a long-term change prediction unit that predicts, based on a classification result of the vegetation by the vegetation classification unit and the remote sensing data, long-term wide-area fluctuation that is range fluctuation of the vegetation in a predetermined long-term time sequence;
a short-term change prediction unit that predicts, based on the classification result of the vegetation by the vegetation classification unit and the remote sensing data, short-term wide-area fluctuation that is range fluctuation of the vegetation in a predetermined short-term time sequence;
a risk determination unit that determines, based on a prediction result by the long-term change prediction unit and a prediction result by the short-term change prediction unit, a risk due to contact of the vegetation and the facility; and
a visualization unit that visualizes a determination result of the risk by the risk determination unit.

2. The vegetation management system according to claim 1, further comprising a tree height estimation unit that estimates, for each kind of vegetation classified by the vegetation classification unit, tree height that is height of a tree crown of the vegetation photographed in the remote sensing data, wherein
in addition to the prediction of the long-term wide-area fluctuation, the long-term change prediction unit predicts, based on an estimation result of the tree height by the tree height estimation unit, long-term tree height fluctuation that is tree height fluctuation of the vegetation in the long-term time sequence,
in addition to the prediction of the short-term wide-area fluctuation, the short-term change prediction unit predicts, based on the estimation result of the height of the tree crown by the tree height estimation unit, short-term tree height fluctuation that is tree height fluctuation of the vegetation in the short-term time sequence, and
the risk determination unit determines the risk due to the contact of the vegetation and the facility based on prediction results of the long-term wide-area fluctuation and the long-term tree height fluctuation by the long-term change prediction unit and prediction results of the short-term wide-area fluctuation and the short-term tree height fluctuation by the short-term change prediction unit.

3. The vegetation management system according to claim 2, further comprising a maintenance instruction unit that instructs, based on a determination result by the risk determination unit and data for visualization generated by the visualization unit, maintenance work for eliminating the risk due to the contact of the vegetation and the facility.

4. The vegetation management system according to claim 2, wherein the determination of the risk by the risk determination unit includes:
growth risk determination for, for each of the long-term time sequence and the short-term time sequence in which at least parts of periods overlap, evaluating a risk that the vegetation and the facility come into contact in a wide area because of the range fluctuation of the vegetation predicted by the long-term wide-area fluctuation and the short-term wide-area fluctuation;
contact risk determination for, for each of the long-term time sequence and the short-term time sequence for which the growth risk determination is performed, evaluating a detailed risk that the vegetation and the facility come into contact because of the tree height fluctuation of the vegetation predicted in the long-term tree height fluctuation and the short-term tree height fluctuation; and
damage risk determination for predicting, based on an evaluation result of the contact risk determination, a damage risk of the facility according to a simulation.

5. The vegetation management system according to claim 4, wherein, in the growth risk determination, the risk determination unit constructs, using a prediction result of the short-term wide-area fluctuation, a short-term growth risk model for estimating a risk due to fall-down of the vegetation due to meteorological fluctuation and constructs, using a prediction result of the long-term wide-area fluctuation, a long-term growth risk model for estimating a risk due to growth of the vegetation.

6. The vegetation management system according to claim 4, wherein the risk determination unit performs the growth risk determination using a two-dimensional model and, when contact of the vegetation and the facility is predicted by the determination, performs the contact risk determination and the damage risk determination for a predicted contact part using a three-dimensional model.

7. The vegetation management system according to claim 3, further comprising a data acquisition unit that acquires input data including a plurality of remote sensing data in a time sequence in past, vegetation information data concerning a physiological characteristic of the vegetation, geographical data including information concerning positions and shapes of the vegetation and the facility, environmental data concerning an environment of a region, and management data concerning management and security of the facility and stores the input data in a database, wherein
the vegetation classification unit classifies, using the input data other than the management data, the vegetation photographed in the remote sensing data and generates a vegetation classification map on which a result of the classification is mapped,
the tree height estimation unit creates, using the input data other than the management data, a tree height estimation model for estimating height of the tree crown with respect to the vegetation classified by the vegetation classification unit and generates a tree height map on which an estimation result of tree height by the tree height estimation model is mapped,
the long-term change prediction unit generates, using the remote sensing data in a long-term time sequence in the past, the classification result of the vegetation by the vegetation classification unit, the physiological characteristic indicated by the vegetation information data for the vegetation, the geographical data, and the environmental data, a long-term time sequence change prediction model for predicting remote sensing data in a long-term time sequence in future, generates a long-term vegetation wide-area map on which a prediction result by the long-term time sequence change prediction model is mapped, further estimates tree height using the tree height estimation model for the long-term vegetation wide-area map, and generates a long-term tree height prediction map on which a result of the estimation is mapped,
the short-term change prediction unit generates, using the remote sensing data in a short-term time sequence in the past, the classification result of the vegetation by the vegetation classification unit, the physiological characteristic indicated by the vegetation information data for the vegetation, the geographical data, and the environmental data, a short-term time sequence change prediction model for predicting remote sensing data in a short-term time sequence in future, generates a short-term vegetation wide-area map on which a prediction result by the short-term time sequence change prediction model is mapped, further estimates tree height using the tree height estimation model for the short-term vegetation wide-area map, and generates a short-term tree height prediction map on which a result of the estimation is mapped,
the risk determination unit determines a risk due to contact of the vegetation and the facility using generated objects by the long-term change prediction unit and the short-term change prediction unit and the geographical data, and
the maintenance instruction unit determines instruction content of the maintenance work based on a determination result by the risk determination unit, data for visualization generated by the visualization unit, and the management data and outputs the instruction content.

8. The vegetation management system according to claim 1, wherein, in the predetermined long-term time sequence, a time interval in a degree in which fluctuation in the vegetation due to growth of a tree can be checked is designated and, in the predetermined short-term time sequence, a time interval in a degree in which fluctuation in the vegetation due to meteorological fluctuation can be checked is designated.

9. A vegetation management method by a vegetation management system that analyzes, using remote sensing data obtained by photographing, with remote sensing, a facility and vegetation that are analysis targets, a risk that the vegetation comes into contact with the facility, the vegetation management method comprising:
a vegetation classification step in which the vegetation management system classifies the vegetation photographed in the remote sensing data;
a long-term change prediction step in which the vegetation management system predicts, based on a classification result of the vegetation by the vegetation classification step and the remote sensing data, long-term wide-area fluctuation that is range fluctuation of the vegetation in a predetermined long-term time sequence;
a short-term change prediction step in which the vegetation management system predicts, based on the classification result of the vegetation by the vegetation classification step and the remote sensing data, short-term wide-area fluctuation that is range fluctuation of the vegetation in a predetermined short-term time sequence;
a risk determination step in which the vegetation management system determines, based on a prediction result by the long-term change prediction step and a prediction result by the short-term change prediction step, a risk due to contact of the vegetation and the facility; and
a visualization step in which the vegetation management system visualizes a determination result of the risk by the risk determination step.

10. The vegetation management method according to claim 9, further comprising a tree height estimation step in which the vegetation management system estimates, for each kind of vegetation classified in the vegetation classification step, tree height that is height of a tree crown of the vegetation photographed in the remote sensing data, wherein
in the long-term change prediction step, in addition to the prediction of the long-term wide-area fluctuation, the vegetation management system predicts, based on an estimation result of the tree height by the tree height estimation step, long-term tree height fluctuation that is tree height fluctuation of the vegetation in the long-term time sequence,
in the short-term change prediction step, in addition to the prediction of the short-term wide-area fluctuation, the vegetation management system predicts, based on the estimation result of the height of the tree crown by the tree height estimation step, short-term tree height fluctuation that is tree height fluctuation of the vegetation in the short-term time sequence, and
in the risk determination step, the vegetation management system determines the risk due to the contact of the vegetation and the facility based on prediction results of the long-term wide-area fluctuation and the long-term tree height fluctuation by the long-term change prediction step and prediction results of the short-term wide-area fluctuation and the short-term tree height fluctuation by the short-term change prediction step.

11. The vegetation management method according to claim 10, further comprising a maintenance instruction step in which the vegetation management system instructs, based on a determination result in the risk determination step and data for visualization generated in the visualization step, maintenance work for eliminating the risk due to the contact of the vegetation and the facility.

## Patentansprüche

1. Vegetationsverwaltungssystem, das unter Verwendung von Fernerkundungsdaten, die durch Fotografieren einer Anlage und einer Vegetation erhalten werden, die Analyseziele sind, ein Risiko analysiert, dass die Vegetation mit der Anlage in Kontakt kommt, wobei das Vegetationsverwaltungssystem Folgendes umfasst:
eine Vegetationsklassifizierungseinheit, die die in den Fernerkundungsdaten fotografierte Vegetation klassifiziert;
eine Langzeitänderungsvorhersageeinheit, die basierend auf einem Klassifizierungsergebnis der Vegetation durch die Vegetationsklassifizierungseinheit und den Fernerkundungsdaten eine langfristige großflächige Schwankung vorhersagt, die eine Bereichsschwankung der Vegetation in einer vorbestimmten Langzeit-Zeitsequenz ist;
eine Kurzzeitänderungsvorhersageeinheit, die basierend auf dem Klassifizierungsergebnis der Vegetation durch die Vegetationsklassifizierungseinheit und den Fernerkundungsdaten eine kurzfristige großflächige Schwankung vorhersagt, die eine Bereichsschwankung der Vegetation in einer vorbestimmten Kurzzeit-Zeitsequenz ist;
eine Risikobestimmungseinheit, die basierend auf einem Vorhersageergebnis durch die Langzeitänderungsvorhersageeinheit und einem Vorhersageergebnis durch die Kurzzeitänderungsvorhersageeinheit ein Risiko aufgrund eines Kontakts der Vegetation und der Anlage bestimmt; und
eine Visualisierungseinheit, die ein Bestimmungsergebnis des Risikos durch die Risikobestimmungseinheit visualisiert.

2. Vegetationsverwaltungssystem nach Anspruch 1, ferner umfassend eine Baumhöhenschätzeinheit, die für jede von der Vegetationsklassifizierungseinheit klassifizierte Art von Vegetation eine Baumhöhe schätzt, die die Höhe einer Baumkrone der in den Fernerkundungsdaten fotografierten Vegetation ist, wobei die Langzeitänderungsvorhersageeinheit zusätzlich zu der Vorhersage der langfristigen großflächigen Schwankung eine langfristige Baumhöhenschwankung, die eine Baumhöhenschwankung der Vegetation in der Langzeit-Zeitsequenz ist, basierend auf einem Schätzergebnis der Baumhöhe durch die Baumhöhenschätzeinheit vorhersagt,
die Kurzzeitänderungsvorhersageeinheit zusätzlich zu der Vorhersage der kurzfristigen großflächigen Schwankung eine kurzfristige Baumhöhenschwankung, die eine Baumhöhenschwankung der Vegetation in der Kurzzeit-Zeitsequenz ist, basierend auf dem Schätzergebnis der Höhe der Baumkrone durch die Baumhöhenschätzeinheit vorhersagt, und
die Risikobestimmungseinheit das Risiko aufgrund des Kontakts der Vegetation und der Anlage basierend auf Vorhersageergebnissen der langfristigen großflächigen Schwankung und der langfristigen Baumhöhenschwankung durch die Langzeitänderungsvorhersageeinheit und Vorhersageergebnissen der kurzfristigen großflächigen Schwankung und der kurzfristigen Baumhöhenschwankung durch die Kurzzeitänderungsvorhersageeinheit bestimmt.

3. Vegetationsverwaltungssystem nach Anspruch 2, ferner umfassend eine Wartungsanweisungseinheit, die basierend auf einem Bestimmungsergebnis durch die Risikobestimmungseinheit und Daten zur Visualisierung, die durch die Visualisierungseinheit erzeugt werden, Wartungsarbeiten zum Beseitigen des Risikos aufgrund des Kontakts der Vegetation und der Einrichtung anweist.

4. Vegetationsverwaltungssystem nach Anspruch 2, wobei die Bestimmung des Risikos durch die Risikobestimmungseinheit Folgendes beinhaltet:
Wachstumsrisikobestimmung für jede der Langzeit-Zeitsequenz und der Kurzzeit-Zeitsequenz, in denen sich zumindest Teile von Zeiträumen überlappen, zum Beurteilen eines Risikos, dass die Vegetation und die Anlage aufgrund der durch die langfristige großflächige Schwankung und die kurzfristige großflächige Schwankung vorhergesagten Bereichsschwankung der Vegetation auf einer großen Fläche in Kontakt kommen;
Kontaktrisikobestimmung für jede der Langzeit-Zeitsequenz und der Kurzzeit-Zeitsequenz, für die die Wachstumsrisikobestimmung durchgeführt wird, zum Beurteilen eines detaillierten Risikos, dass die Vegetation und die Anlage aufgrund der in der langfristigen Baumhöhenschwankung und der kurzfristigen Baumhöhenschwankung vorhergesagten Baumhöhenschwankung der Vegetation in Kontakt kommen; und
Schadensrisikobestimmung zum Vorhersagen, basierend auf einem Beurteilungsergebnis der Kontaktrisikobestimmung, eines Schadensrisikos der Anlage gemäß einer Simulation.

5. Vegetationsverwaltungssystem nach Anspruch 4, wobei die Risikobestimmungseinheit bei der Wachstumsrisikobestimmung unter Verwendung eines Vorhersageergebnisses der kurzfristigen großflächigen Schwankung ein Kurzzeit-Wachstumsrisikomodell zum Schätzen eines Risikos aufgrund eines Abfallens der Vegetation aufgrund meteorologischer Schwankungen aufbaut und unter Verwendung eines Vorhersageergebnisses der langfristigen großflächigen Schwankung ein Langzeit-Wachstumsrisikomodell zum Schätzen eines Risikos aufgrund des Wachstums der Vegetation aufbaut.

6. Vegetationsverwaltungssystem nach Anspruch 4, wobei die Risikobestimmungseinheit die Wachstumsrisikobestimmung unter Verwendung eines zweidimensionalen Modells durchführt und, wenn der Kontakt der Vegetation und der Anlage durch die Bestimmung vorhergesagt wird, die Kontaktrisikobestimmung und die Schadensrisikobestimmung für einen vorhergesagten Kontaktteil unter Verwendung eines dreidimensionalen Modells durchführt.

7. Vegetationsverwaltungssystem nach Anspruch 3, ferner umfassend eine Datenerfassungseinheit, die Eingabedaten erfasst, die eine Mehrzahl von Fernerkundungsdaten in einer Zeitsequenz in der Vergangenheit, Vegetationsinformationsdaten bezüglich einer physiologischen Eigenschaft der Vegetation, geografische Daten, die Informationen bezüglich Positionen und Formen der Vegetation und der Anlage beinhalten, Umgebungsdaten bezüglich einer Umgebung einer Region und Verwaltungsdaten bezüglich der Verwaltung und Sicherheit der Anlage beinhalten, und die Eingabedaten in einer Datenbank speichert, wobei
die Vegetationsklassifizierungseinheit unter Verwendung der Eingabedaten außer den Verwaltungsdaten die in den Fernerkundungsdaten fotografierte Vegetation klassifiziert und eine Vegetationsklassifizierungskarte erzeugt, auf die ein Ergebnis der Klassifizierung abgebildet wird,
die Baumhöhenschätzeinheit unter Verwendung der Eingabedaten außer den Verwaltungsdaten ein Baumhöhenschätzmodell zum Schätzen der Höhe der Baumkrone in Bezug auf die durch die Vegetationsklassifizierungseinheit klassifizierte Vegetation erstellt und eine Baumhöhenkarte erzeugt, auf die ein Schätzergebnis der Baumhöhe durch das Baumhöhenschätzmodell abgebildet wird,
die Langzeitänderungsvorhersageeinheit unter Verwendung der Fernerkundungsdaten in einer Langzeit-Zeitsequenz in der Vergangenheit, des Klassifizierungsergebnisses der Vegetation durch die Vegetationsklassifizierungseinheit, der physiologischen Eigenschaft, die durch die Vegetationsinformationsdaten für die Vegetation angegeben wird, der geografischen Daten und der Umgebungsdaten ein Langzeit-Zeitsequenz-Änderungsvorhersagemodell zum Vorhersagen von Fernerkundungsdaten in einer Langzeit-Zeitsequenz in der Zukunft erzeugt, eine großflächige Langzeit-Vegetationskarte erzeugt, auf die ein Vorhersageergebnis durch das Langzeit-Zeitsequenz-Änderungsvorhersagemodell abgebildet wird, ferner die Baumhöhe unter Verwendung des Baumhöhenschätzmodells für die großflächige Langzeit-Vegetationskarte schätzt und eine Langzeit-Baumhöhenvorhersagekarte erzeugt, auf die ein Ergebnis der Schätzung abgebildet wird,
die Kurzzeitänderungsvorhersageeinheit unter Verwendung der Fernerkundungsdaten in einer Kurzzeit-Zeitsequenz in der Vergangenheit, des Klassifizierungsergebnisses der Vegetation durch die Vegetationsklassifizierungseinheit, der physiologischen Eigenschaft, die durch die Vegetationsinformationsdaten für die Vegetation angegeben wird, der geografischen Daten und der Umgebungsdaten ein Kurzzeit-Zeitsequenz-Änderungsvorhersagemodell zum Vorhersagen von Fernerkundungsdaten in einer Kurzzeit-Zeitsequenz in der Zukunft erzeugt, eine großflächige Kurzzeit-Vegetationskarte erzeugt, auf die ein Vorhersageergebnis durch das Kurzzeit-Zeitsequenz-Änderungsvorhersagemodell abgebildet wird, ferner die Baumhöhe unter Verwendung des Baumhöhenschätzmodells für die großflächige Kurzzeit-Vegetationskarte schätzt und eine Kurzzeit-Baumhöhenvorhersagekarte erzeugt, auf die ein Ergebnis der Schätzung abgebildet wird,
die Risikobestimmungseinheit ein Risiko aufgrund eines Kontakts der Vegetation und der Anlage unter Verwendung von erzeugten Objekten durch die Langzeitänderungsvorhersageeinheit und die Kurzzeitänderungsvorhersageeinheit und der geografischen Daten bestimmt, und
die Wartungsanweisungseinheit Anweisungsinhalt der Wartungsarbeiten basierend auf einem Bestimmungsergebnis durch die Risikobestimmungseinheit, Daten zur Visualisierung, die durch die Visualisierungseinheit erzeugt werden, und den Verwaltungsdaten bestimmt und den Anweisungsinhalt ausgibt.

8. Vegetationsverwaltungssystem nach Anspruch 1, wobei in der vorbestimmten Langzeit-Zeitsequenz ein Zeitintervall in einem Ausmaß festgelegt wird, in dem eine Schwankung in der Vegetation aufgrund von Wachstum eines Baums überprüft werden kann, und in der vorbestimmten Kurzzeit-Zeitsequenz ein Zeitintervall in einem Ausmaß festgelegt wird, in dem eine Schwankung in der Vegetation aufgrund meteorologischer Schwankungen überprüft werden kann.

9. Vegetationsverwaltungsverfahren durch ein Vegetationsverwaltungssystem, das unter Verwendung von Fernerkundungsdaten, die durch Fotografieren einer Anlage und einer Vegetation erhalten werden, die Analyseziele sind, ein Risiko analysiert, dass die Vegetation mit der Anlage in Kontakt kommt, wobei das Vegetationsverwaltungsverfahren Folgendes umfasst:
einen Vegetationsklassifizierungsschritt, in dem das Vegetationsverwaltungssystem die in den Fernerkundungsdaten fotografierte Vegetation klassifiziert;
einen Langzeitänderungsvorhersageschritt, in dem das Vegetationsverwaltungssystem basierend auf einem Klassifizierungsergebnis der Vegetation durch den Vegetationsklassifizierungsschritt und den Fernerkundungsdaten eine langfristige großflächige Schwankung vorhersagt, die eine Bereichsschwankung der Vegetation in einer vorbestimmten Langzeit-Zeitsequenz ist;
einen Kurzzeitänderungsvorhersageschritt, in dem das Vegetationsverwaltungssystem basierend auf dem Klassifizierungsergebnis der Vegetation durch den Vegetationsklassifizierungsschritt und den Fernerkundungsdaten eine kurzfristige großflächige Schwankung vorhersagt, die eine Bereichsschwankung der Vegetation in einer vorbestimmten Kurzzeit-Zeitsequenz ist;
einen Risikobestimmungsschritt, in dem das Vegetationsverwaltungssystem basierend auf einem Vorhersageergebnis durch den Langzeitänderungsvorhersageschritt und einem Vorhersageergebnis durch den Kurzzeitänderungsvorhersageschritt ein Risiko aufgrund eines Kontakts der Vegetation und der Anlage bestimmt; und
einen Visualisierungsschritt, in dem das Vegetationsverwaltungssystem ein Bestimmungsergebnis des Risikos durch den Risikobestimmungsschritt visualisiert.

10. Vegetationsverwaltungsverfahren nach Anspruch 9, ferner umfassend einen Baumhöhenschätzschritt, in dem das Vegetationsverwaltungssystem für jede in dem Vegetationsklassifizierungsschritt klassifizierte Art von Vegetation eine Baumhöhe schätzt, die die Höhe einer Baumkrone der in den Fernerkundungsdaten fotografierten Vegetation ist, wobei
in dem Langzeitänderungsvorhersageschritt das Vegetationsverwaltungssystem zusätzlich zu der Vorhersage der langfristigen großflächigen Schwankung eine langfristige Baumhöhenschwankung, die eine Baumhöhenschwankung der Vegetation in der Langzeit-Zeitsequenz ist, basierend auf einem Schätzergebnis der Baumhöhe durch den Baumhöhenschätzschritt vorhersagt,
in dem Kurzzeitänderungsvorhersageschritt das Vegetationsverwaltungssystem zusätzlich zu der Vorhersage der kurzfristigen großflächigen Schwankung eine kurzfristige Baumhöhenschwankung, die eine Baumhöhenschwankung der Vegetation in der Kurzzeit-Zeitsequenz ist, basierend auf dem Schätzergebnis der Höhe der Baumkrone durch den Baumhöhenschätzschritt vorhersagt, und
in dem Risikobestimmungsschritt das Vegetationsverwaltungssystem das Risiko aufgrund des Kontakts der Vegetation und der Anlage basierend auf Vorhersageergebnissen der langfristigen großflächigen Schwankung und der langfristigen Baumhöhenschwankung durch den Langzeitänderungsvorhersageschritt und Vorhersageergebnissen der kurzfristigen großflächigen Schwankung und der kurzfristigen Baumhöhenschwankung durch den Kurzzeitänderungsvorhersageschritt bestimmt.

11. Vegetationsverwaltungsverfahren nach Anspruch 10, ferner umfassend einen Wartungsanweisungsschritt, in dem das Vegetationsverwaltungssystem basierend auf einem Bestimmungsergebnis durch den Risikobestimmungsschritt und Daten zur Visualisierung, die in dem Visualisierungsschritt erzeugt werden, Wartungsarbeiten zum Beseitigen des Risikos aufgrund des Kontakts der Vegetation und der Anlage anweist.

## Revendications

1. Système de gestion de végétation qui analyse, en utilisant des données de télédétection obtenues en photographiant, avec télédétection, une installation et une végétation qui sont des cibles d'analyse, un risque que la végétation entre en contact avec l'installation, le système de gestion de végétation comprenant :
une unité de classification de végétation qui classifie la végétation photographiée dans les données de télédétection ;
une unité de prédiction de changement à long terme qui prédit, sur la base d'un résultat de classification de la végétation par l'unité de classification de végétation et des données de télédétection, une fluctuation de grande zone à long terme qui est une fluctuation d'étendue de la végétation dans une séquence temporelle à long terme prédéterminée ;
une unité de prédiction de changement à court terme qui prédit, sur la base du résultat de classification de la végétation par l'unité de classification de végétation et des données de télédétection, une fluctuation de grande zone à court terme qui est une fluctuation d'étendue de la végétation dans une séquence temporelle à court terme prédéterminée ;
une unité de détermination de risque qui détermine, sur la base d'un résultat de prédiction par l'unité de prédiction de changement à long terme et d'un résultat de prédiction par l'unité de prédiction de changement à court terme, un risque dû à un contact de la végétation et de l'installation ; et
une unité de visualisation qui visualise un résultat de détermination du risque par l'unité de détermination de risque.

2. Système de gestion de végétation selon la revendication 1, comprenant en outre une unité d'estimation de hauteur d'arbre qui estime, pour chaque type de végétation classifiée par l'unité de classification de végétation, une hauteur d'arbre qui est une hauteur d'un houppier de la végétation photographiée dans les données de télédétection, dans lequel
en plus de la prédiction de la fluctuation de grande zone à long terme, l'unité de prédiction de changement à long terme prédit, sur la base d'un résultat d'estimation de la hauteur d'arbre par l'unité d'estimation de hauteur d'arbre, une fluctuation de hauteur d'arbre à long terme qui est une fluctuation de hauteur d'arbre de la végétation dans la séquence temporelle à long terme,
en plus de la prédiction de la fluctuation de grande zone à court terme, l'unité de prédiction de changement à court terme prédit, sur la base du résultat d'estimation de la hauteur du houppier par l'unité d'estimation de hauteur d'arbre, une fluctuation de hauteur d'arbre à court terme qui est une fluctuation de hauteur d'arbre de la végétation dans la séquence temporelle à court terme, et
l'unité de détermination de risque détermine le risque dû au contact de la végétation et de l'installation sur la base de résultats de prédiction de la fluctuation de grande zone à long terme et de la fluctuation de hauteur d'arbre à long terme par l'unité de prédiction de changement à long terme et de résultats de prédiction de la fluctuation de grande zone à court terme et de la fluctuation de hauteur d'arbre à court terme par l'unité de prédiction de changement à court terme.

3. Système de gestion de végétation selon la revendication 2, comprenant en outre une unité d'instruction d'entretien qui donne l'instruction, sur la base d'un résultat de détermination par l'unité de détermination de risque et de données pour visualisation générées par l'unité de visualisation, d'un travail d'entretien pour éliminer le risque dû au contact de la végétation et de l'installation.

4. Système de gestion de végétation selon la revendication 2, dans lequel la détermination du risque par l'unité de détermination de risque inclut :
une détermination de risque d'accroissement pour, pour chacune de la séquence temporelle à long terme et de la séquence temporelle à court terme dans lesquelles au moins des parties de périodes se chevauchent, évaluer un risque que la végétation et l'installation entrent en contact dans une grande zone en raison de la fluctuation d'étendue de la végétation prédite par la fluctuation de grande zone à long terme et la fluctuation de grande zone à court terme ;
une détermination de risque de contact pour, pour chacune de la séquence temporelle à long terme et de la séquence temporelle à court terme pour lesquelles la détermination de risque d'accroissement est réalisée, évaluer un risque détaillé que la végétation et l'installation entrent en contact en raison de la fluctuation de hauteur d'arbre de la végétation prédite dans la fluctuation de hauteur d'arbre à long terme et la fluctuation de hauteur d'arbre à court terme ; et
une détermination de risque de dégâts pour prédire, sur la base d'un résultat d'évaluation de la détermination de risque de contact, un risque de dégâts de l'installation selon une simulation.

5. Système de gestion de végétation selon la revendication 4, dans lequel, dans la détermination de risque d'accroissement, l'unité de détermination de risque construit, en utilisant un résultat de prédiction de la fluctuation de grande zone à court terme, un modèle de risque d'accroissement à court terme pour estimer un risque dû à une chute de la végétation due à une fluctuation météorologique et construit, en utilisant un résultat de prédiction de la fluctuation de grande zone à long terme, un modèle de risque d'accroissement à long terme pour estimer un risque dû un accroissement de la végétation.

6. Système de gestion de végétation selon la revendication 4, dans lequel l'unité de détermination de risque réalise la détermination de risque d'accroissement en utilisant un modèle bidimensionnel et, lorsqu'un contact de la végétation et de l'installation est prédit par la détermination, réalise la détermination de risque de contact et la détermination de risque de dégâts pour une partie de contact prédite en utilisant un modèle tridimensionnel.

7. Système de gestion de végétation selon la revendication 3, comprenant en outre une unité d'acquisition de données qui acquiert des données d'entrée incluant une pluralité de données de télédétection dans une séquence temporelle dans le passé, des données d'informations de végétation concernant une caractéristique physiologique de la végétation, des données géographiques incluant des informations concernant des positions et des formes de la végétation et de l'installation, des données environnementales concernant un environnement d'une région, et des données de gestion concernant une gestion et une sécurité de l'installation, et stocke les données d'entrée dans une base de données, dans lequel
l'unité de classification de végétation classifie, en utilisant les données d'entrée autres que les données de gestion, la végétation photographiée dans les données de télédétection et génère une carte de classification de végétation sur laquelle un résultat de la classification est cartographié,
l'unité d'estimation de hauteur d'arbre crée, en utilisant les données d'entrée autres que les données de gestion, un modèle d'estimation de hauteur d'arbre pour estimer une hauteur du houppier en ce qui concerne la végétation classifiée par l'unité de classification de végétation et génère une carte de hauteur d'arbre sur laquelle un résultat d'estimation de hauteur d'arbre par le modèle d'estimation de hauteur d'arbre est cartographié,
l'unité de prédiction de changement à long terme génère, en utilisant les données de télédétection dans une séquence temporelle à long terme dans le passé, le résultat de classification de la végétation par l'unité de classification de végétation, la caractéristique physiologique indiquée par les données d'informations de végétation pour la végétation, les données géographiques, et les données environnementales, un modèle de prédiction de changement de séquence temporelle à long terme pour prédire des données de télédétection dans une séquence temporelle à long terme dans le futur, génère une carte de grande zone de végétation à long terme sur laquelle un résultat de prédiction par le modèle de prédiction de changement de séquence temporelle à long terme est cartographié, estime en outre une hauteur d'arbre en utilisant le modèle d'estimation de hauteur d'arbre pour la carte de grande zone de végétation à long terme, et génère une carte de prédiction de hauteur d'arbre à long terme sur laquelle un résultat de l'estimation est cartographié,
l'unité de prédiction de changement à court terme génère, en utilisant les données de télédétection dans une séquence temporelle à court terme dans le passé, le résultat de classification de la végétation par l'unité de classification de végétation, la caractéristique physiologique indiquée par les données d'informations de végétation pour la végétation, les données géographiques, et les données environnementales, un modèle de prédiction de changement de séquence temporelle à court terme pour prédire des données de télédétection dans une séquence temporelle à court terme dans le futur, génère une carte de grande zone de végétation à court terme sur laquelle un résultat de prédiction par le modèle de prédiction de changement de séquence temporelle à court terme est cartographié, estime en outre une hauteur d'arbre en utilisant le modèle d'estimation de hauteur d'arbre pour la carte de grande zone de végétation à court terme, et génère une carte de prédiction de hauteur d'arbre à court terme sur laquelle un résultat de l'estimation est cartographié,
l'unité de détermination de risque détermine un risque dû à un contact de la végétation et de l'installation en utilisant des objets générés, par l'unité de prédiction de changement à long terme et l'unité de prédiction de changement à court terme, et les données géographiques, et
l'unité d'instruction d'entretien détermine un contenu d'instruction du travail d'entretien sur la base d'un résultat de détermination par l'unité de détermination de risque, de données pour visualisation générées par l'unité de visualisation, et des données de gestion, et produit en sortie le contenu d'instruction.

8. Système de gestion de végétation selon la revendication 1, dans lequel, dans la séquence temporelle à long terme prédéterminée, un intervalle temporel à un degré auquel une fluctuation de la végétation due à un accroissement d'un arbre peut être contrôlée est désigné et, dans la séquence temporelle à court terme prédéterminée, un intervalle temporel à un degré auquel une fluctuation de la végétation due à une fluctuation météorologique peut être contrôlée est désigné.

9. Procédé de gestion de végétation par un système de gestion de végétation qui analyse, en utilisant des données de télédétection obtenues en photographiant, avec télédétection, une installation et une végétation qui sont des cibles d'analyse, un risque que la végétation entre en contact avec l'installation, le procédé de gestion de végétation comprenant :
une étape de classification de végétation dans laquelle le système de gestion de végétation classifie la végétation photographiée dans les données de télédétection ;
une étape de prédiction de changement à long terme dans laquelle le système de gestion de végétation prédit, sur la base d'un résultat de classification de la végétation par l'étape de classification de végétation et des données de télédétection, une fluctuation de grande zone à long terme qui est une fluctuation d'étendue de la végétation dans une séquence temporelle à long terme prédéterminée ;
une étape de prédiction de changement à court terme dans laquelle le système de gestion de végétation prédit, sur la base du résultat de classification de la végétation par l'étape de classification de végétation et des données de télédétection, une fluctuation de grande zone à court terme qui est une fluctuation d'étendue de la végétation dans une séquence temporelle à court terme prédéterminée ;
une étape de détermination de risque dans laquelle le système de gestion de végétation détermine, sur la base d'un résultat de prédiction par l'étape de prédiction de changement à long terme et d'un résultat de prédiction par l'étape de prédiction de changement à court terme, un risque dû à un contact de la végétation et de l'installation ; et
une étape de visualisation dans laquelle le système de gestion de végétation visualise un résultat de détermination du risque par l'étape de détermination de risque.

10. Procédé de gestion de végétation selon la revendication 9, comprenant en outre une étape d'estimation de hauteur d'arbre dans laquelle le système de gestion de végétation estime, pour chaque type de végétation classifiée dans l'étape de classification de végétation, une hauteur d'arbre qui est une hauteur d'un houppier de la végétation photographiée dans les données de télédétection, dans lequel
dans l'étape de prédiction de changement à long terme, en plus de la prédiction de la fluctuation de grande zone à long terme, le système de gestion de végétation prédit, sur la base d'un résultat d'estimation de la hauteur d'arbre par l'étape d'estimation de hauteur d'arbre, une fluctuation de hauteur d'arbre à long terme qui est une fluctuation de hauteur d'arbre de la végétation dans la séquence temporelle à long terme,
dans l'étape de prédiction de changement à court terme, en plus de la prédiction de la fluctuation de grande zone à court terme, le système de gestion de végétation prédit, sur la base du résultat d'estimation de la hauteur du houppier par l'étape d'estimation de hauteur d'arbre, une fluctuation de hauteur d'arbre à court terme qui est une fluctuation de hauteur d'arbre de la végétation dans la séquence temporelle à court terme, et dans l'étape de détermination de risque, le système de gestion de végétation détermine le risque dû au contact de la végétation et de l'installation sur la base de résultats de prédiction de la fluctuation de grande zone à long terme et de la fluctuation de hauteur d'arbre à long terme par l'étape de prédiction de changement à long terme et de résultats de prédiction de la fluctuation de grande zone à court terme et de la fluctuation de hauteur d'arbre à court terme par l'étape de prédiction de changement à court terme.

11. Procédé de gestion de végétation selon la revendication 10, comprenant en outre une étape d'instruction d'entretien dans laquelle le système de gestion de végétation donne l'instruction, sur la base d'un résultat de détermination dans l'étape de détermination de risque et de données pour visualisation générées dans l'étape de visualisation, d'un travail d'entretien pour éliminer le risque dû au contact de la végétation et de l'installation.
